# EUROPEAN PATENT APPLICATION

(11) **EP 4 700 020 A1**
(43) Date of publication of application: **25.02.2026**
(21) Application number: 24792034.1
(22) Date of filing: 17.04.2024
(51) Int. Cl.: C07D 401/14, C07D 401/12, C07D 403/12, C07D 403/14, A61K 31/4439, A61P 29/00, A61P 17/06, A61P 17/00, A61P 19/02, A61P 25/00

(54) **HETEROCYCLIC COMPOUND AS TYK2 INHIBITOR, AND SYNTHESIS AND USE THEREOF**

(30) Priority: 21.04.2023 CN 202310452583; 28.08.2023 CN 202311090183
(71) Applicant: Zhejiang Wenda Pharmaceutical Technology Co., Ltd., Hangzhou, Zhejiang 310018 (CN)
(72) Inventor: WANG, Nenghui, Hangzhou, Zhejiang 310018 (CN)
(74) Representative: Dehns
(86) International application number: PCT/CN2024/088280
(87) International publication number: WO 2024/217449

(57) **Abstract**

Provided in the present invention are a heterocyclic compound as a TYK2 inhibitor, and synthesis, and use thereof.. Specifically, provided is a compound or a pharmaceutically acceptable salt thereof. The compound is as represented by formula A, and each group is as defined herein.

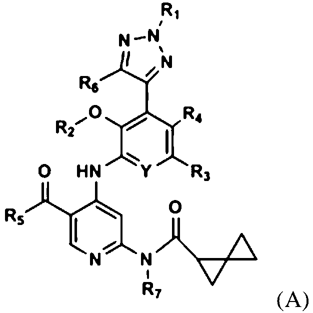

## Description

### Technical field

The present invention belongs to the field of pharmaceuticals and medical aesthetics, specifically, relates to a heterocyclic compound as a TYK2 inhibitor, and synthesis and use thereof.

### Background

Janus kinase (JAK) is an intracellular non receptor tyrosine kinase that mediates the signaling transduction and activation of various cytokines. The JAK kinase family is divided into four subtypes: JAK1, JAK2, JAK3, and TYK2. Each subtype mediates different types of cytokine signaling pathways. JAK1, JAK2, and TYK2 are expressed in various tissues and cells in the human body, while JAK3 is mainly expressed in hematopoietic tissue cells. A common feature of cytokine receptors is that the receptor itself does not have kinase activity, but the intracellular segment of the receptor has a binding site for the tyrosine kinase JAK. After the cytokine receptor binding to its ligand, receptor-coupled JAK activates, which in turn phosphorylates the receptor. The phosphorylated tyrosine site can bind to the SH2 domain-containing STAT protein, resulting in STAT being recruited to the receptor and phosphorylated via the JAK, and then the dimerization of phosphotyrosine-mediated STAT. The activated STAT dimer transfers to the nucleus of the cell and activates the transcription of its target genes, thus regulating multiple functions such as growth, activation, and differentiation of various cells.

TYK2 is the earliest subtype discovered in the JAK family, mediating the functions of cytokines such as IFN- a, IL-6, IL-10, IL-12, and IL-23. Studies have shown that TYK2 deletion mutations can effectively inhibit the occurrence of immune diseases such as allergies, autoimmune diseases, and inflammation. IL-23 plays a crucial role in the occurrence and development of psoriasis. Recent studies have shown that the pathogenesis of psoriasis is the secretion of IL-23 caused by the activation of antigen presenting cells APC by endogenous unknown antigen. IL-23 activates Th17 cells to secrete cytokines such as IL-17, inducing keratinocyte differentiation and secretion of IL-23, further stimulating inflammation and keratinocyte proliferation to produce psoriasis. TYK2 and JAK2 jointly mediate the downstream signaling pathway of IL-23, while inhibition of JAK2 may lead to anemia and other blood related side effects. Therefore, inhibition of TYK2 is a good strategy for inhibiting IL-23 signaling pathway.

Early TYK2 inhibitor such as Tofacitinib is JAK non-selective inhibitors and is the first oral JAK inhibitor with significant inhibitory activity against JAK1, 2, and 3 subtypes. The inhibition of other subtypes such as JAK1, JAk2, and JAk3 increases the efficacy of Tofacitinib, but also brings serious side effects, including infections, tuberculosis, tumors, anemia, liver damage, and increased cholesterol. Due to the correlation between JAK2 activity and red blood cell differentiation and lipid metabolism processes, some of above-mentioned side effects such as anemia are considered being related to the insufficient selectivity of Tofacitinib to JAK2, and are caused by the non-selective inhibition of the drug. There are no TYK2-selective inhibitors currently on the market, and early JAK inhibitors acts mainly by competing for the binding of the kinase structural domain to ATP, and thus generally had poor selectivity.

TYK2 is also associated with a number of cancers, such as acute lymphoblastic leukemia (T-ALL) where abnormal cell survival is associated with TYK2 activation. Knockout experiments showed that 88% of T-ALL cell lines and 63% of T-ALL cells from patients were TYK2-dependent, and thus TYK2 is an oncogene for T-ALL. TYK2 selective inhibitor NDI-031301 was able to induce apoptosis to inhibit the growth of human T-ALL cell lines, and also showed excellent safety and efficacy in a mouse model bearing KOPT-K1T-ALL tumor cells, demonstrating the prospect of TYK2 selective inhibitors in the treatment of T-ALL.

Given the favorable efficacy of JAK non-selective inhibitors and the severe side effects associated with multiple targets, it has great potential in clinical application to develop a safer TYK2-selective inhibitor for the treatment of a wide range of TYK2-associated autoimmune and inflammation-related disorders including psoriasis, lupus erythematosus, inflammatory bowel disease, psoriatic arthritis, arthritis, vasculitis, fibrosis, dermatitis, skin aging, cephalitis, lupus nephritis, neurogenic inflammation, different types of multiple sclerosis (including optic neuritis, ophthalmoneuromyelitis), chronic inflammatory demyelinating polyneuropathy, Parkinson, dementia, Amyotrophic Lateral Sclerosis, myasthenia gravis, mental disease, schizophrenia, epilepsy, Spinal injury, Sleep disorder, cerebral injury, stroke, Neuropsychiatric Systemic Lupus Erythematosus, diabetic encephalopathy, Sepsis associated encephalopathy, Central Nervous System Neoplasms, Huntington's disease, Neurological syndrome after surgery, pain, itching, depressive disorder, Hypersomnia, hydrocephalus, Ankylosing Spondylitis, respiratory disease, diabetes, Inflammatory eye disease, hepatitis, cardiovascular disease, systemic sclerosis, organ transplantation, alopecia areata, acne, eczema, leucoderma, sjogren syndrome, viral inflammation, some cancers and the like.

Therefore, there is an urgent need in this field for the development of structurally novel, highly selective and more effective brain-penetrating TYK2 inhibitors, particularly those suitable for use in central nervous system diseases.

### Summary of the invention

The purpose of the present invention is to provide a class of structurally novel brain-penetrating TYK2 inhibitors with high safety and excellent potency.

In the first aspect of the present invention, provided is a compound or a pharmaceutically acceptable salt thereof, wherein the compound is of formula A1, 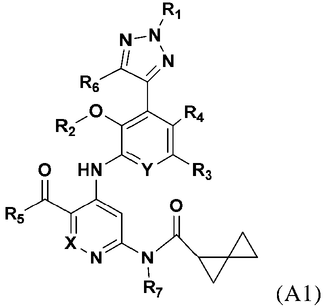 wherein,
X is N or CR₈; R₈ is selected from the group consisting of: H, and optionally substituted C₁₋₄alkyl;
Y, R₁, R₂, R₃, R₄, R₅, R₆, R₇, R₉ and "optionally substituted" are as defined in Formula A.

In another preferred embodiment, provided is a compound or a pharmaceutically acceptable salt thereof, wherein the compound is of formula A; 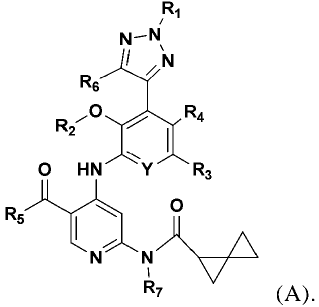
Y is N or CR₉;
R₁ is selected from the group consisting of: H, and optionally substituted C₁-₄alkyl;
R₂ is selected from the group consisting of: H, and optionally substituted C₁₋₄alkyl;
R₃ and R₄ are each independently selected from the group consisting of: H, halogen and optionally substituted C₁₋₄alkyl;
R₅ is selected from the group consisting of: H, and optionally substituted C₁₋₆alkyl;
R₆ is selected from the group consisting of: H, and optionally substituted C₁₋₄alkyl;
R₇ is selected from the group consisting of: H, and optionally substituted C₁₋₄alkyl;
R₉ is selected from the group consisting of: H, and optionally substituted C₁₋₄alkyl;
the "optionally substituted" means that the group is unsubstituted or is substituted with one or more substituents selected from the group consisting of: deuterium halogen, C₁₋₄ alkyl, and C₁₋₄ haloalkyl.

In another preferred embodiment, R₄ is selected from the group consisting of: H, methyl, and ethyl.

In another preferred embodiment, R₄ is H.

In another preferred embodiment, R₅ is C₁₋₆alkyl.

In another preferred embodiment, R₅ is selected from the group consisting of: methyl, ethyl, n-propyl, and n-butyl.

In another preferred embodiment, R₅ is ethyl.

In another preferred embodiment, R₆ is selected from the group consisting of: H, methyl, and ethyl.

In another preferred embodiment, R₆ is H.

In another preferred embodiment, R₆ is selected from the group consisting of: H, methyl, and ethyl.

In another preferred embodiment, R₆ is H.

In another preferred embodiment, R₇ is selected from the group consisting of: H, methyl, and ethyl.

In another preferred embodiment, R₇ is H.

In another preferred embodiment, the compound is of formula A1-S or formula A1-R.

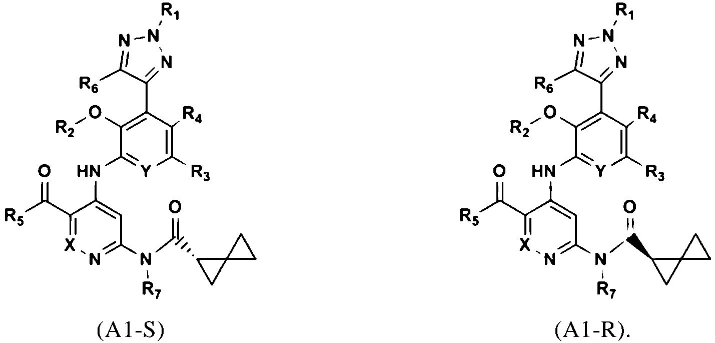

In another preferred embodiment, the compound is of formula A-S or formula A-R.

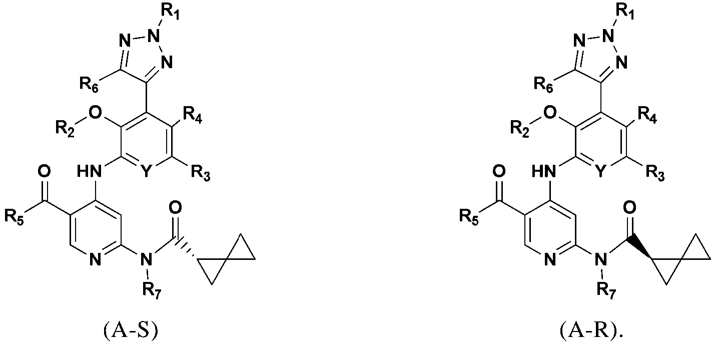

In another preferred embodiment, the compound is of formula A-S (i.e., S-configuration).

In another preferred embodiment, the compound is of formula I1

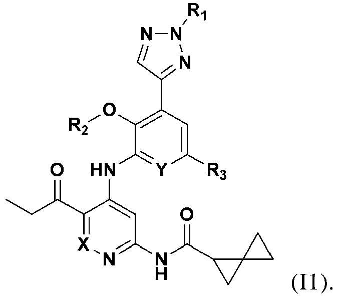

In another preferred embodiment, the compound is of formula I

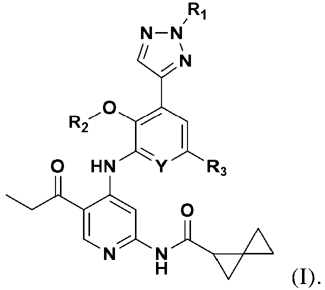

In another preferred embodiment, R₈ and R₉ are each independently selected from the group consisting of: H, methyl, and ethyl.

In another preferred embodiment, R₈ is H.

In another preferred embodiment, R₉ is H.

In another preferred embodiment, X is N or CH.

In another preferred embodiment, X is CRs.

In another preferred embodiment, X is CH.

In another preferred embodiment, Y is N or CH.

In another preferred embodiment, Y is N.

In another preferred embodiment, R₁ is selected from the group consisting of: C₁₋₄alkyl, and deuterated C₁₋₄alkyl.

In another preferred embodiment, R₁ is selected from the group consisting of: methyl, ethyl, deuterated methyl, and deuterated ethyl.

In another preferred embodiment, R₁ is selected from the group consisting of: -CH₃, and -CD₃.

In another preferred embodiment, R₂ is selected from the group consisting of: C₁₋₄alkyl, and deuterated C₁₋₄alkyl.

In another preferred embodiment, R₂ is selected from the group consisting of: methyl, ethyl, deuterated methyl, and deuterated ethyl.

In another preferred embodiment, R₂ is selected from the group consisting of: -CH₃, and -CD₃.

In another preferred embodiment, R₃ is selected from the group consisting of: H, and C₁₋₄alkyl.

In another preferred embodiment, R₃ is selected from the group consisting of: H, methyl, and ethyl.

In another preferred embodiment, the compound is of formula I1-S or formula I1-R.

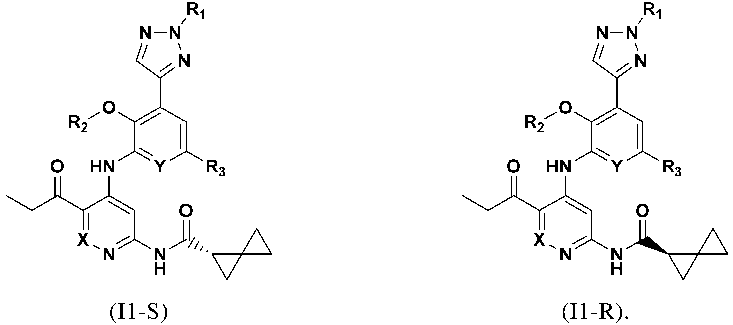

In another preferred embodiment, the compound is of formula I-S or formula I-R.

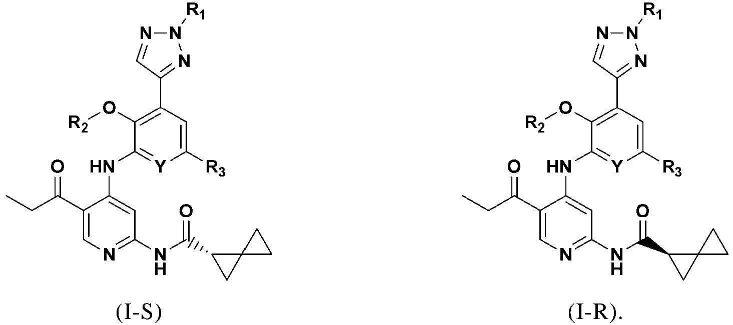

In another preferred embodiment, the compound is of formula I-S (i.e., S-configuration).

In another preferred embodiment, X, Y, R₁, R₂, R₃, R₄, R₅, R₆, R₇, R₈ and R₉ are each independently the corresponding groups in specific compounds shown in examples, Table A and Table B.

In another preferred embodiment, the compound is a compound selected from Table A or table B, or a pharmaceutically acceptable salt thereof:

**Table A**

| | | |
|---|---|---|
| 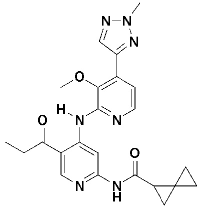 1 | 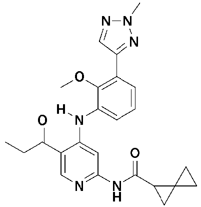 2 | 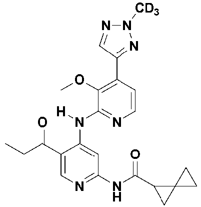 3 |
| 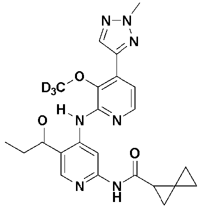 4 | 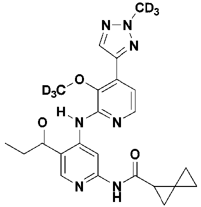 5 | 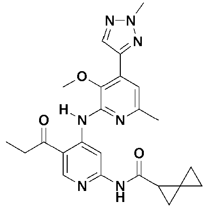 6 |

**Table B**

| | | | |
|---|---|---|---|
| 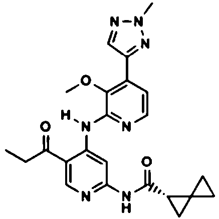 1S | 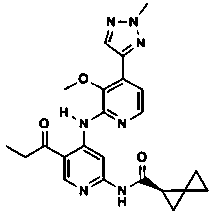 1R | 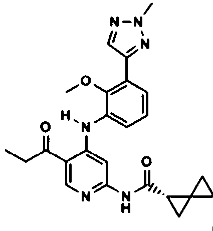 2S | 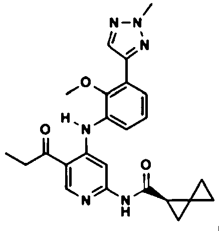 2R |
| 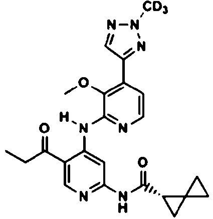 3S | 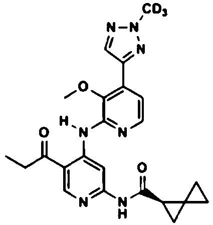 3R | 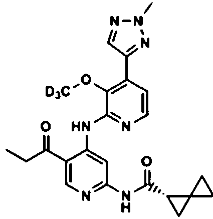 4S | 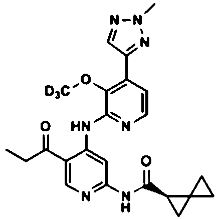 4R |
| 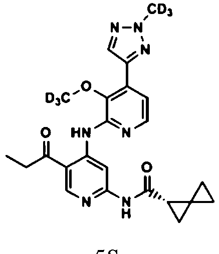 5S | 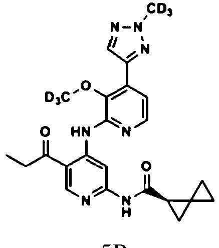 5R | 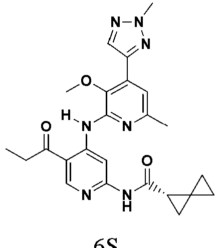 6S | 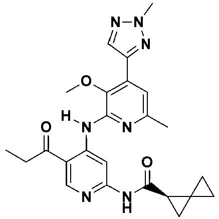 6R |

In another preferred embodiment, the compound is Compound 1S, 2S, 3S, 4S, 5S, or 6S in Table B.

In another preferred embodiment, the compound is Compound 4S in Table B.

In the second aspect of the present invention, provided is a pharmaceutical composition, comprising:
(i) the compound according to the first aspect or the pharmaceutically acceptable salt thereof, and (ii) pharmaceutically acceptable carriers or excipients.

In the third aspect of the present invention, provided is a use of the compound according to the first aspect or the pharmaceutical composition according to the second aspect in the preparation of (i) a drug for treating or preventing a TYK2-mediated disease and/or (ii) a TYK2 inhibitor.

In another preferred embodiment, the TYK2-mediated diseases comprise: psoriasis, lupus erythematosus, inflammatory bowel disease, psoriatic arthritis, arthritis, vasculitis, fibrosis, dermatitis, skin aging, cephalitis, lupus nephritis, neurogenic inflammation, multiple sclerosis (including optic neuritis, ophthalmoneuromyelitis), chronic inflammatory demyelinating polyneuropathy, Parkinson, dementia, Amyotrophic Lateral Sclerosis, myasthenia gravis, mental disease, schizophrenia, epilepsy, Spinal injury, Sleep disorder, cerebral injury, stroke, Neuropsychiatric Systemic Lupus Erythematosus, diabetic encephalopathy, Sepsis associated encephalopathy, Central Nervous System Neoplasms, Huntington's disease, Neurological syndrome after surgery, pain, itching, depressive disorder, Hypersomnia, hydrocephalus, Ankylosing Spondylitis, respiratory disease, diabetes, Inflammatory eye disease, hepatitis, cardiovascular disease, systemic sclerosis, organ transplantation, alopecia areata, acne, eczema, leucoderma, sjogren syndrome, viral inflammation, cancer, or combinations thereof.

In the fourth aspect of the present invention, provided is a method for inhibiting TYK2, comprising:
contacting the subject with the compound according to the first aspect, thereby inhibiting the activity of TYK2 in the subject.

In another preferred embodiment, the subject is cell.

In another preferred embodiment, the method is *in vitro* and non-therapeutic.

In the fifth aspect of the present invention, provided is a method for inhibiting the expression of pSTAT5 in a cell, comprising:
contacting the subject with the compound according to the first aspect, thereby inhibiting the expression of pSTAT5 in the cell.

In another preferred embodiment, the method is *in vitro* and non-therapeutic.

In the sixth aspect of the present invention, provided is a method for treating or preventing a TYK2-mediated disease, comprising a step of:
administering a safe and effective amount of the compound according to the first aspect or the pharmaceutical composition according to the second aspect to a human in need thereof, thereby treating or preventing the TYK2-mediated disease.

**In** another preferred embodiment, the TYK2-mediated diseases comprise: psoriasis, lupus erythematosus, inflammatory bowel disease, psoriatic arthritis, arthritis, vasculitis, fibrosis, dermatitis, skin aging, cephalitis, lupus nephritis, neurogenic inflammation, multiple sclerosis (including optic neuritis, ophthalmoneuromyelitis), chronic inflammatory demyelinating polyneuropathy, Parkinson, dementia, Amyotrophic Lateral Sclerosis, myasthenia gravis, mental disease, schizophrenia, epilepsy, Spinal injury, Sleep disorder, cerebral injury, stroke, Neuropsychiatric Systemic Lupus Erythematosus, diabetic encephalopathy, Sepsis associated encephalopathy, Central Nervous System Neoplasms, Huntington's disease, Neurological syndrome after surgery, pain, itching, depressive disorder, Hypersomnia, hydrocephalus, Ankylosing Spondylitis, respiratory disease, diabetes, Inflammatory eye disease, hepatitis, cardiovascular disease, systemic sclerosis, organ transplantation, alopecia areata, acne, eczema, leucoderma, sjogren syndrome, viral inflammation, cancer, or combinations thereof.

**In** the seventh aspect of the present invention, provided is a preparation method of a compound, wherein the compound is of compound I-S, 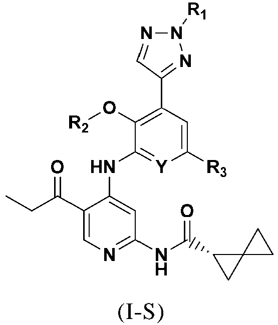 and the preparation method comprises a step of:
reacting an intermediate of formula II-S with an intermediate of formula III, thereby obtaining the compound of formula I-S; 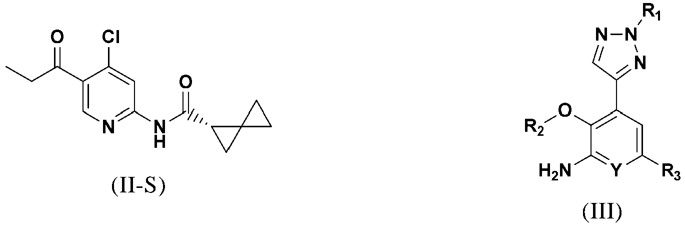
in each formula, Y, R₁, R₂ and R₃ are as defined in formula I.

In the eighth aspect of the present invention, provided is an intermediate of formula II-S,

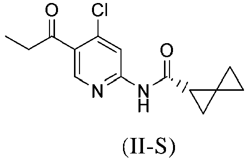

It should be understood that within the scope of the present invention, the above technical features of the present invention and the technical features specifically described in the following (eg, embodiments) can be combined with each other, thereby forming a new or preferred technical solution. Due to space limitations, it will not be repeated herein.

### Description of the drawings

FIG. 1A and FIG. 1B show the results of animal weight and clinical scores in Test Example 3.
FIG. 2A and FIG. 2B show the TNF-α and IFN-γ levels in serum in Test Example 3.

### Embodiments for carrying out the invention

After extensive and in-depth research, the inventors unexpectedly found that a class of compounds with specific novel structure (e.g., 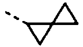 ), in particular the S-configuration of the compound, have excellent inhibitory activity against TYK2 and lower toxicity. In addition, the inventors have overcome the challenges of chiral synthesis of compounds containing a structure of 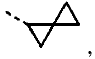 thereby avoiding the use of chiral chromatography, and thus providing a more industrially accessible class of compounds with superior inhibitory activity against TYK2 and lower toxicity. Based on this, the inventors completed the present invention.

### TERMS

As used herein, the term "halogen" refers to F, Cl, Br or I. Correspondingly, "halo" means hydrogen atom in the group is substituted with F, Cl, Br, or I.

Unless otherwise stated, the term "alkyl", by itself or as a part of another substituent, means a straight or branched chain hydrocarbon radical, having a specific number of carbon atoms (i.e. C₁₋₆ means 1 - 6 carbons). Preferably, alkyl has 1 - 4 carbons i.e., C1-4alkyl, more preferably has 1 - 3 carbons i.e., C₁₋₃alkyl. Examples of alkyl groups include methyl, ethyl, n-propyl, isopropyl, n-butyl, t-butyl, *iso*-butyl, *sec*-butyl, n-pentyl, n-hexyl, and the like.

As used herein, the term "containing", "comprising" or "including" means that the various components can be used together in the mixture or composition of the present invention. Therefore, the terms "mainly consisting of ..." and "consisting of ..." are within the scope of the term "comprising".

As used herein, the term "deuterated" means one or more hydrogen atoms in the group are substituted by deuterium; preferably, means that all hydrogen atoms in the group are substituted by deuterium.

As used herein, the term "pharmaceutically acceptable" component(s) refer to a substance suitable for use in human and/or animals without causing excessive adverse side reactions (such as toxicity, stimulation and allergic reaction), i.e. a substance with reasonable benefit/risk ratio.

Unless otherwise specified, all compounds present in the present invention are intended to include all possible optical isomers, such as single chiral compounds or mixtures of various chiral compounds (i.e. racemes). Among all compounds of the present invention, each chiral carbon atom may optionally be of the R or S configuration, or a mixture of the R and S configurations.

Certain compounds of the present disclosure possess asymmetric carbon atoms (optical centers) or double bonds; the racemates, diastereomers, geometric isomers, regioisomers and individual isomers (e.g., separate enantiomers) are all intended to be encompassed within the scope of the present disclosure. When the compound provided herein has an identified stereochemistry (indicated as R or S, or with dashed or wedge bond designations), those compounds will be understood by one of skill in the art to be substantially free of other isomers (e.g., at least 80%, 90%, 95%, 98%, 99%, and up to 100% free of the other isomer).

The compounds of the present disclosure may also contain unnatural proportions of isotope atomic isotopes at one or more isotopic atoms that constitute such compounds. The unnatural proportions of certain isotope can be defined as the amount from the naturally found amount of the atom discussed to 100% amount of that atom. For example, the compounds may incorporate radioactive isotopes, such as tritium (³H), iodine-125 (¹²⁵I) or carbon-14 (¹⁴C), or non-radioactive isotopes, such as deuterium (²H) or carbon-13 (¹³C). Such isotopic variants may provide additional uses in addition to those described in this application. For instance, isotopic variants of the compounds of the disclosure may find additional utility, including but not limited to, as diagnostic and/or imaging reagents, or as cytotoxic/radiotoxic therapeutic agents. Additionally, isotopic variants of the compounds of the disclosure can have altered pharmacokinetic and pharmacodynamic characteristics which can contribute to enhanced safety, tolerability or efficacy during treatment. All isotopic variants of the compounds of the present disclosure, whether radioactive or not, should be encompassed within the scope of the present disclosure.

### Active Ingredients

As used herein, the term "compound of the invention" or "compound of the invention" refers to the compound of formula (A) or (I). This term also includes various crystalline forms, pharmaceutically acceptable salts, hydrates or solvates of the compound of formula (A) or formula (I).

Wherein, the term "pharmaceutically acceptable salts" refers to salts that are formed of the compound of the invention and acids or bases, and suitable for use as a drug. Pharmaceutically acceptable salts include inorganic salts and organic salts. A group of preferred salts are salts formed of the compound of the present invention and acids. Acids suitable for forming salts include, but are not limited to, inorganic acids such as hydrochloric acid, hydrobromic acid, hydrofluoric acid, sulfuric acid, nitric acid, phosphoric acid and the like; organic acids such as methanoic acid, acetic acid, trifluoroacetic acid, propionic acid, oxalic acid, malonic acid, succinic acid, fumaric acid, maleic acid, lactic acid, malic acid, tartaric acid, citric acid, bitter acid, benzoic acid, methanesulfonic acid, ethanesulfonic acid, p-toluenesulfonic acid, benzenesulfonic acid, naphthalene sulfonic acid, and the like; and amino acids such as proline, phenylalanine, aspartic acid, glutamic acid and the like. Another preferred class of salts are salts formed of the compounds of the invention with bases, such as alkali metal salts (such as sodium or potassium salts), alkaline earth metal salts (such as magnesium or calcium salts), ammonium salts (such as lower grades alkanol ammonium salts and other pharmaceutically acceptable amine salts), such as methylamine salt, ethylamine salt, propylamine salt, dimethylamine salt, trimethylamine salt, diethylamine salt, triethylamine salt, tert-butylamine salt, ethylenediamine salt, hydroxyethylamine salt, dihydroxyethylamine salt, trihydroxyethylamine salt, and amine salts formed from morpholine, piperazine, and lysine, respectively.

The term "solvate" refers to complexes formed of the compound of the present invention and solvent molecules in any specific ratio. "Hydrate" refers to a complex formed by the coordination of the compound of the invention with water.

Moreover, the compound of the present invention further comprises prodrugs of the compound of formula (A) or formula (I). The term "prodrug", including itself, is biologically active or non-active, and is a class of compounds that readily undergo metabolism or chemical reaction in the human body and convert to the compound of formula (A) or formula (I) or the salts thereof or solutions containing the compound of formula (A) or formula (I) when being administered in a suitable way. The prodrugs include (but are not limited to) the carboxylate, carbonate, phosphate, nitrate, sulfate, sulfone ester, sulfoxide ester, amino compound, carbamate, azo compound, phosphamide, glucoside, ether, acetal of the compound, etc..

### Preparation method

Other parts herein provides a more specific description of the preparation methods for the compounds having a structure of formula (A) or formula (I) of the present invention, but these specific methods do not pose any limitations to the present invention. The compound of the present invention can also be conveniently prepared by combining various synthesis methods described in this specification or known in the art, and such combinations can be easily carried out by those skilled in this field to which the present invention belongs.

### Pharmaceutical compositions and administration method

Because the compounds of the invention have excellent activity against TYK2 (selective inhibitory activity), the compounds of the invention and various crystal forms, pharmaceutically acceptable inorganic or organic salts, hydrates or solvates thereof, and pharmaceutical compositions containing the compound of the present invention as main active ingredients can be used in the treatment, prevention and alleviation of diseases mediated by TYK2 kinase (i.e., TYK2-mediated disease). According to the prior art, the compounds of the invention can be used to treat the following diseases: psoriasis, lupus erythematosus, inflammatory bowel disease, psoriatic arthritis, arthritis, vasculitis, fibrosis, dermatitis, skin aging, cephalitis, lupus nephritis, neurogenic inflammation, different types of multiple sclerosis (including optic neuritis, ophthalmoneuromyelitis), chronic inflammatory demyelinating polyneuropathy, Parkinson, dementia, Amyotrophic Lateral Sclerosis, myasthenia gravis, mental disease, schizophrenia, epilepsy, Spinal injury, Sleep disorder, cerebral injury, stroke, Neuropsychiatric Systemic Lupus Erythematosus, diabetic encephalopathy, Sepsis associated encephalopathy, Central Nervous System Neoplasms, Huntington's disease, Neurological syndrome after surgery, pain, itching, depressive disorder, Hypersomnia, hydrocephalus, Ankylosing Spondylitis, respiratory disease, diabetes, Inflammatory eye disease, hepatitis, cardiovascular disease, systemic sclerosis, organ transplantation, alopecia areata, acne, eczema, leucoderma, sjogren syndrome, viral inflammation, some cancers, and the like.

The pharmaceutical composition of the invention comprises the compound of the present invention or the pharmaceutically acceptable salts thereof in a safe and effective dosage range, and pharmaceutically acceptable excipients or carriers. In which, "safe and effective amount" refers to that the amount of the compound is sufficient to significantly improve the condition without causing serious side effects. Generally, the pharmaceutical composition contains 1-500mg of the compound of the present invention/dose, more preferably, 1-200mg of the compound of the present invention/dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of the pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc..

There is no special limitation on the administration mode for the compound or pharmaceutical compositions of the present invention, and the representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. **In** these solid dosage forms, the active compounds are mixed with at least one conventional inert excipient (or carrier), such as sodium citrate or Ca(H₂PO₄)₂, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or mixtures thereof. **In** capsules, tablets, and pills, the dosage form may also include a buffer.

Solid dosage forms such as tablets, sugar pills, capsules, pills, and granules can be prepared using coating and shell materials, such as casings and other well-known materials in the art. They can contain an opaque agent. The active compounds or the compounds in the compositions can be released in a delayed mode in a given portion of the digestive tract. Examples of the embedding component that can be used are polymeric substances and waxes. If necessary, the active compound can also form microcapsules with one or more of the aforementioned excipients.

Liquid dosage forms for oral administration include pharmaceutically acceptable emulsions, solutions, suspensions, syrups or tinctures. **In** addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

**In** addition to these inert diluents, the composition can also include additives such as wetting agents, emulsifiers and suspending agents, sweeteners, corrigents, and spices.

**In** addition to active compounds, suspensions can include suspending agents, such as ethoxylated isooctadecanol, polyoxyethylene sorbitol and dehydrated sorbitol esters, microcrystalline cellulose, methanol aluminum and agar, or mixtures of these substances.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols, and their suitable mixtures.

The dosage forms of the compounds of the present invention used for local administration include ointments, powders, patches, sprays, and inhalants. The active ingredients are mixed under sterile conditions with physiologically acceptable carriers and any preservatives, buffers, or propellants that may be necessary.

The compound of the present invention can be administered separately or in combination with other pharmaceutically acceptable compounds.

When the pharmaceutical composition is used, a safe and effective amount of the compound of the present invention is administered to a mammal (such as a human) in need of treatment, wherein the dosage at the time of administration is the pharmaceutically effective dosage, for people having a body weight of 60kg, the daily dose is usually 1~ 500mg, preferably 1~200mg. Of course, the specific dosage should also consider factors such as the route of administration and the patient's health status, which are within the skill range of a skilled physician.

### The main advantages of the present invention include:

1. The compound of the present invention (in particular, the compound of the invention with S configuration) has excellent inhibitory activity against the TYK2.
2. The compound of the present invention has excellent inhibitory activity against the expression of pSTATS.
3. The compound of the present invention has excellent brain permeability (ability to penetrate the blood-brain barrier).

The present invention was further described hereafter in combination with specific embodiments. It should be understood that these examples are only used to illustrate the and not to limit the scope of the invention. The experimental methods without specific conditions in the following examples generally follow the conventional conditions or the conditions suggested by the manufacturer. Unless otherwise stated, percentages and parts are percentages by weight and parts by weight.

### Preparation Example

### Example 1

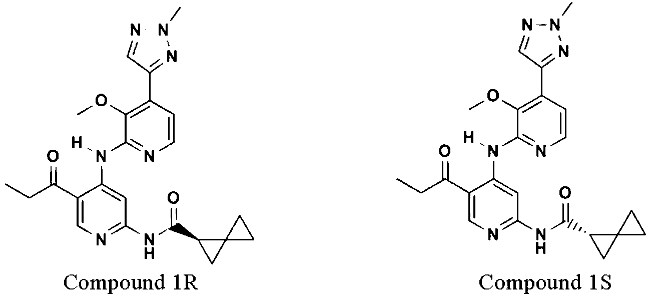

### STEP A

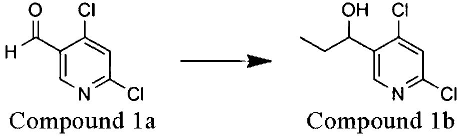

Ethyl magnesium bromide (24ml, 71.6 mmol) was added into a three-necked flask containing 200 ml tetrahydrofuran at 0 °C. A solution of Compound 1a (6 g, 34.1 mmol) in tetrahydrofuran was slowly added dropwise under nitrogen protection at 0 °C. The mixture was stirred at room temperature for 5 hours. LCMS was used to monitor the reaction. After the reaction was complete, the reaction was quenched with saturated ammonium chloride solution (100ml), diluted with water (100mL), extracted with ethyl acetate (100mL x 3), washed with saturated sodium chloride aqueous solution (100mL), dried over anhydrous Na2SO4, and filtered. The filtrate was collected, concentrated under reduced pressure to obtain a yellow liquid, which was separated by column chromatography (petroleum ether/ethyl acetate=4/1) to obtain compound 1b (3.55 g, 50.57%) as a yellow liquid. LCMS: 206.0, 208.0[M+H].

### STEP B

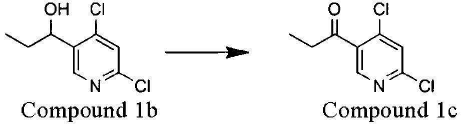

Compound 1b (3.55 g, 17.2 mmol), and Dess-Martin oxidizer (14.59 g, 34.4 mmol) were sequentially added to a 250 ml round-bottomed flask containing 100 mL of anhydrous dichloromethane and the mixture was stirred for 16 h at room temperature. The reaction was monitored by LCMS. After the reaction was complete, the pH of the reaction solution was adjusted to 7-8 by adding saturated aqueous sodium bicarbonate solution. The mixture was extracted with dichloromethane (100 mL × 3). The extractions were combined, and washed with saturated saline (100 ml); the organic phase was collected, dried over anhydrous Na2SO4, concentrated under reduced pressure, and separated by column chromatography (petroleum ether/ethyl acetate, 4/1) to give compound 1c (3.2 g, 91.16%) as a yellow solid. LCMS: 204.0, 206.0 [M+H]+.

### STEP C

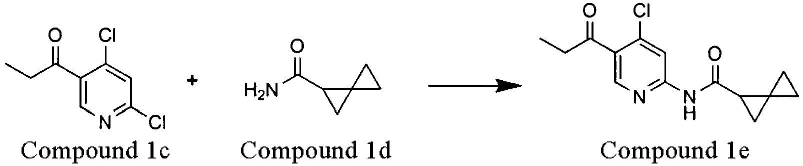

Compound 1c (300 mg, 1.47 mmol) and Compound 1d (245.1 mg, 2.21 mmol) were dissolved in dioxane (10 mL). The solution was successively added with Pd2(dba)3 (238.35 mg, 0.29 mmol), cesium carbonate (1.44 g, 4.41 mmol) and 1, 1'-bis(dicyclohexylphosphino)ferrocene (dcpf, 335.5 mg, 0.59 mmol). The mixture was stirred at 80°C for 3 hours under nitrogen protection. The reaction was monitored by LCMS. After the completion of reaction, the reaction was concentrated under reduced pressure, diluted by adding water (20 mL), and extracted with ethyl acetate (20 mL x 3). The extractions were combined, washed with saturated saline (20 mL), dried over anhydrous sodium sulfate, and concentrated under reduced pressure. The residue was separated by column chromatograph (petroleum ether/ethyl acetate, 3/1) to obtain Compound 1e (289 mg,70.56%) as a yellow solid. 1H NMR (400 MHz, CD3OD) δ 8.59 (s, 1H), 8.33 (s, 1H), 3.04 (q, J = 7.2 Hz, 2H), 2.27 (dd, J = 7.4, 4.2 Hz, 1H), 1.58 (t, J = 3.9 Hz, 1H), 1.47 (dd, J = 7.4, 3.6 Hz, 1H), 1.19 (t, J = 7.2 Hz, 3H), 1.04-0.94 (m, 3H), 0.92-0.87 (m, 1H). LCMS: 279.1, 281.1[M+H]+.

### STEP D

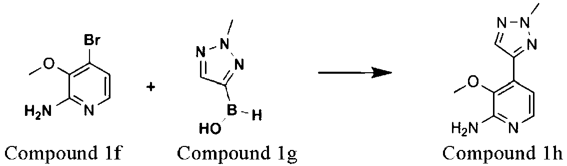

Compound 1f (800 mg, 3.94 mmol) and Compound 1g (500 mg, 3.94 mmol) were dissolved in a mixed solvent of dioxane/water (20 mL/4 mL). The solution was sequentially added with Pd(dppf)Cl2-CH2Cl2 (160 mg, 0.20 mmol) and potassium carbonate (1.09 g, 7.88 mmol), and the mixture was stirred for 16 h at 110 °C under nitrogen protection. The reaction was monitored by LCMS. After the completion of reaction, the reaction was diluted by adding water (50 mL) and extracted with ethyl acetate (50 mL x 3). The extractions were combined, washed with saturated saline (50 mL), dried over anhydrous sodium sulfate, concentrated under reduced pressure, and separated by flash preparative chromatography (dichloromethane/methanol, 15/1) under medium pressure to obtain Compound 1h (550 mg,68.06%) as an off-white solid. 1H NMR (400 MHz, DMSO-d6): δ ppm: 8.16 (s, 1H), 7.73 (d, J = 5.2 Hz, 1H), 6.96 (d, J = 5.6 Hz, 1H), 6.07 (s, 2H), 4.23 (s, 3H), 3.64 (s, 3H). LCMS: 205.9 [M+H]+.

### STEP E

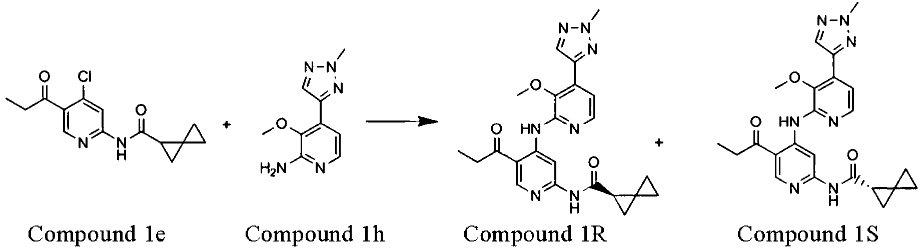

Compound 1e (75 mg, 0.27 mmol) and Compound 1gh (55.2 mg, 0.27 mmol) were dissolved in dioxane (5 mL). The solution was successively added with XantPhos (62.28 mg, 0.11 mmol), cesium carbonate (263 mg, 0.81 mmol) and Pd2 dba)3 (49.2 mg, 0.054 mmol). The mixture was stirred at 115°C under microwave for 2 hours under nitrogen protection. The reaction was monitored by LCMS. After the completion of reaction, the reaction was diluted with water (20 mL) and extracted with ethyl acetate (20 mL x 3). The extractions were combined, washed with saturated saline (20 mL), dried over anhydrous sodium sulfate and concentrated under reduced pressure. The residue was separated by flash preparative chromatography (dichloromethane/methanol, 20/1) under medium pressure to obtain 200 mg crude product. The crude product was separated by preparative HPLC (acetonitrile/water = 55:45; Gemini 5u C18 150 x 21.2 mm) to give 25 mg of product, which was then separated by SFC (Thar Preparative 80, Column: CHIRALPAK IC 250 mm x 20 mm x 5 µm, Modifier: 40% methanol (NH4OH 0.2%)/60% CO2, Total flow rate: 40 g/min, Temperature: 40 °C) to give two isomers: Compound 1R as a white solid (6.1 mg, Rt: 3.65 min), Compound 1S as a white solid (7.4 mg, Rt: 7.82 min) Total yield: 11.26%.

Compound 1R: 1H NMR (400 MHz, DMSO) δ 12.33 (s, 1H), 10.74 (s, 1H), 9.69 (s, 1H), 8.95 (s, 1H), 8.33 (s, 1H), 8.17 (d, J = 5.2 Hz, 1H), 7.47 (d, J = 5.2 Hz, 1H), 4.28 (s,3H), 3.81 (s, 3H), 3.17 (q, J = 7.3 Hz, 2H), 2.42 (dd, J = 7.4, 4.4 Hz, 1H), 1.44 (t, J = 3.8 Hz, 1H), 1.35 (dd, J = 7.4, 3.2 Hz, 1H), 1.14 (t, J = 7.2 Hz, 3H),0.95-0.82 (m, 3H), 0.78 (d, J = 5.4 Hz, 1H). LCMS: 448.2 [M+H]+.

Compound 1S: 1H NMR (400 MHz, DMSO) δ 12.33 (s, 1H), 10.74 (s, 1H), 9.69 (s, 1H), 8.95 (s, 1H), 8.33 (s, 1H), 8.17 (d, J = 5.2 Hz, 1H), 7.47 (d, J = 5.2 Hz, 1H), 4.28 (s,3H), 3.81 (s, 3H), 3.17 (q, J = 7.3 Hz, 2H), 2.42 (dd, J = 7.4, 4.4 Hz, 1H), 1.44 (t, J = 3.8 Hz, 1H), 1.35 (dd, J = 7.4, 3.2 Hz, 1H), 1.14 (t, J = 7.2 Hz, 3H),0.95-0.82 (m, 3H), 0.78 (d, J = 5.4 Hz, 1H). LCMS: 448.2 [M+H]+.

### Example 2

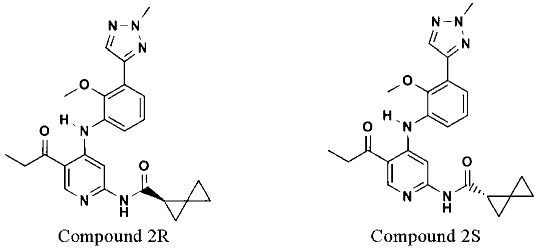

### STEP A

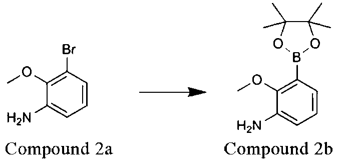

Compound 2a (1.3 g, 6.4 mmol) was dissolved in dioxane (35 mL). The solution was successively added with bis(pinacolato)diboron (4.08 g,16.08mmol), potassium carbonate (0.63g,4.56mmol) and [1,1'-bis(diphenylphosphino)ferrocene]palladium(II) dichloride dichloromethane complex (0.47 g,0.64mmol). The mixture was stirred at 90 °C for 5 hours under nitrogen protection. The reaction was monitored by LCMS. After the completion of reaction, the reaction was cooled to room temperature, and concentrated under reduced pressure to obtain crude product. The crude product was separated by flash preparative chromatography (petroleum ether/ethyl acetate, 2/1) under medium pressure to obtain Compound 2b (1 g,43.4%) as a yellow solid. 1H NMR (400 MHz, DMSO) δ 6.82 - 6.77 (m, 2H), 6.75 (d, J = 4.4 Hz, 1H), 4.82 (s, 2H), 3.63 (s, 3H), 1.28 (s, 12H). LCMS: 250.1 [M+H]+.

### STEP B

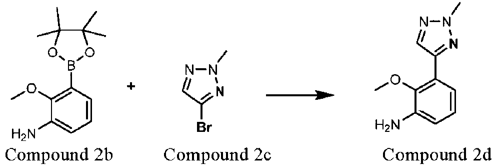

Compound 2b (1.04g, 4.17 mmol) was dissolved in dioxane (16mL) and water (4mL). The solution was successively added with Compound 2c (450 mg, 2.78mmol), tetra(triphenylphosphine)palladium (160.51mg,0.139mmol) and cesium carbonate (1.82g, 5.56mmol). The mixture was stirred at 90 °C for 6 hours under nitrogen protection. The reaction was monitored by LCMS. After the completion of the reaction, the reaction was cooled to room temperature. The reaction solution was concentrated under reduced pressure, the residue was dissolved in ethyl acetate (100mL), and the mixture was washed with water (50ml) and saturated saline solution (50mL). The organic layer was dried over anhydrous sodium sulfate, and concentrated under reduced pressure to obtain crude product. The crude product was separated by flash preparative chromatography (petroleum ether/ethyl acetate, 5/1) under medium pressure to obtain Compound 2d (0.55 g, 63.9%) as a yellow solid. 1H NMR (400 MHz, DMSO-d6): δ ppm 8.47 (s, 1H), 6.96-6.94 (m, 1H), 6.86 (t, J = 8.0 Hz, 1H), 6.75-6.73 (m, 1H), 4.98 (s, 2H), 3.91 (s, 3H), 3.66 (s, 3H). LCMS: 205.1 [M+H]+.

### STEP C

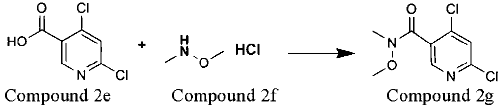

Compound 2e (1 g, 5.2 mmol) was added to a single-necked vial containing 15 ml of N,N-dimethylformamide at room temperature. Compound 2f (0.61 g, 6.24 mmol), N,N-diisopropylethylamine (2.01 g, 15.6 mmol), and 2-(7-azobenzotriazolyl)-N,N,N',N'-tetramethyl urea hexafluorophosphate (2.96g, 7.8mmol) were added under stirring at room temperature. The mixture was stirred at room temperature for 16 h. The reaction was monitored by LCMS. After the completion of the reaction, the reaction solution was poured into 50 mL of ice water, extracted with ethyl acetate (50 mL x 3); the ethyl acetate phase was washed with saturated aqueous sodium chloride solution (20 mL x 3), dried over anhydrous Na2SO4, and filtrated. The filtrate was collected, and concentrated under reduced pressure to obtain the crude yellow liquid, which was separated by column chromatography (ethyl acetate/petroleum ether, 1/5) to obtain the product compound 2g (1.1 g, 80.77%) as a yellow solid. LCMS: 235.0, 237.0 [M+H]+.

### STEP D

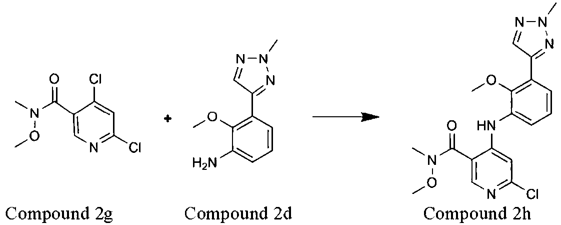

Compound 2g (460 mg, 1.96 mmol) and Compound 2d (400 mg, 1.96 mmol) were dissolved in dry N,N-dimethylformamide (10 mL). Sodium hydride (234mg, 5.88 mmol;60%) was added at 0 °C. The mixture was stirred at room temperature for 16 hours under nitrogen protection. The reaction was monitored by LCMS. After the completion of the reaction, the reaction was quenched by adding water (20 mL) and extracted with ethyl acetate (50 mL × 2). The extractions were combined, washed with saturated saline (30 mL × 2), dried over anhydrous sodium sulfate and concentrated under reduced pressure to obtain crude product. The crude product was separated by flash preparative chromatography (petroleum ether/ethyl acetate, 3/1) under medium pressure to obtain Compound 2h (500 mg,66.5%) as a yellow solid. LCMS: 403.0 [M+H]+.

### STEP E

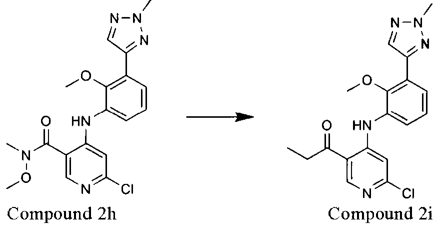

Ethyl magnesium bromide (0.7ml, 2 mmol) was added into a three-necked flask containing 200 ml tetrahydrofuran at 0 °C. A solution of Compound 2h (500 mg, 1.2 mmol) in tetrahydrofuran was slowly added dropwise under nitrogen protection at 0 °C. The mixture was stirred at room temperature for 5 hours. The reaction was monitored by LCMS. After the reaction was complete, the reaction was quenched with saturated ammonium chloride solution (100ml), diluted with water (100mL), extracted with ethyl acetate (100mL x 3), washed with saturated sodium chloride aqueous solution (100mL), dried over anhydrous Na2SO4, and filtered. The filtrate was collected, and concentrated under reduced pressure to obtain a yellow liquid, which was separated by column chromatography (petroleum ether/ethyl acetate, 4/1) to obtain compound 2i (400 mg, 86.2%) as a yellow liquid.

### STEP F

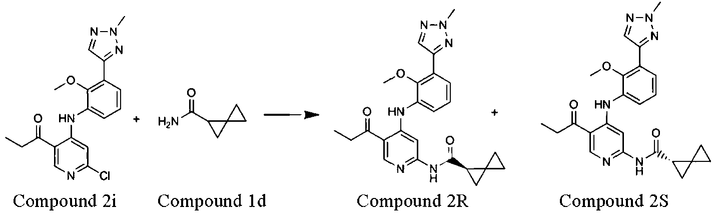

Compound 2i (50 mg, 0.2 mmol) and Compound 1d (22.4 mg, 0.2 mmol) were dissolved in dry dioxane (3 mL). The solution was successively added with 4,5-bis(diphenylphosphino)-9,9-dimethoxyheteroanthracene (15.6mg, 0.0269mmol), cesium carbonate (88.2 mg, 0.269 mmol) and tris(dibenzylideneacetone)dipalladium (10.9 mg, 0.0135 mmol). The mixture was stirred at 115°C under microwave for 1 hour under nitrogen protection. The reaction was monitored by LCMS. After the completion of the reaction, the reaction was cooled to room temperature. The reaction solution was concentrated under reduced pressure, the residue was dissolved in ethyl acetate (50mL), and the mixture was washed with water (30ml) and saturated saline solution (30mL). The organic layer was dried over anhydrous sodium sulfate, and concentrated under reduced pressure to obtain crude product. The crude product was separated by flash preparative chromatography (dichloromethane/methanol, 10/1-5/1) under medium pressure to obtain 40 mg crude product. The crude product was separated by preparative HPLC (acetonitrile/water = 55:45; Gemini 5u C18 150 x 21.2 mm) to give 20 mg of product, which was then separated by SFC (Thar Preparative 80, Column: CHIRALPAK AD-H 250 mm x 20 mm x 5 µm, Modifier: 40% methanol (NH4OH 0.2%)/60% CO2, Total flow rate: 40 g/min, Temperature: 40 °C) to give two isomers: Compound 2R (3.6 mg, Rt: 2.71 min), Compound 2S (3.9 mg, Rt: 3.51 min) Total yield: 12.3%.

Compound 2R: 1H NMR (400 MHz, DMSO) δ 11.09 (s, 1H), 10.74 (s, 1H), 8.88 (s, 1H), 8.13 (s, 1H), 8.05 (s, 1H), 7.72-7.68 (m, 1H), 7.49 (d, J = 7.9 Hz, 1H), 7.31 (t, J = 7.9 Hz, 1H), 4.24 (s, 3H), 3.64 (s, 3H), 3.13 (q, J = 7.1 Hz, 2H), 2.36 (dd, J = 7.4, 4.2 Hz, 1H), 1.36-1.29 (m, 2H), 1.12 (t, J = 7.2 Hz, 3H), 0.88-0.79 (m, 3H), 0.74-0.68 (m, 1H). LCMS: 446.9 [M+H]+.

Compound 2S: 1H NMR (400 MHz, DMSO) δ 11.09 (s, 1H), 10.74 (s, 1H), 8.88 (s, 1H), 8.13 (s, 1H), 8.05 (s, 1H), 7.72-7.68 (m, 1H), 7.49 (d, J = 7.9 Hz, 1H), 7.31 (t, J = 7.9 Hz, 1H), 4.24 (s, 3H), 3.64 (s, 3H), 3.13 (q, J = 7.1 Hz, 2H), 2.36 (dd, J = 7.4, 4.2 Hz, 1H), 1.36-1.29 (m, 2H), 1.12 (t, J = 7.2 Hz, 3H), 0.88 -0.79 (m, 3H), 0.74-0.68 (m, 1H). LCMS: 446.9 [M+H]+.

### Example 3

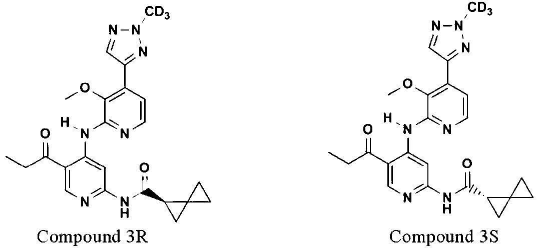

### STEP A

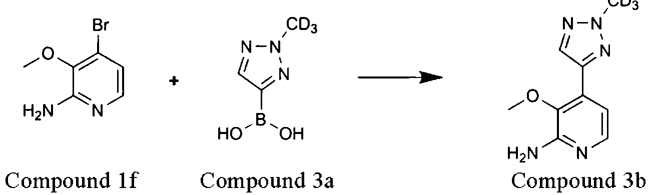

Compound 1f (780 mg, 3.84 mmol) and Compound 3a (500 mg, 3.85 mmol) were dissolved in a mixed solvent of dioxane/water (20 mL/4 mL). The solution was sequentially added with (157 mg, 0.19 mmol) and potassium carbonate (1.06 g, 7.68 mmol), and the mixture was stirred for 16 h at 110 °C under nitrogen protection. The reaction was monitored by LCMS. After the completion of reaction, the reaction was diluted by adding water (50 mL) and extracted with ethyl acetate (50 mL x 3). The extractions were combined, washed with saturated saline (50 mL), dried over anhydrous sodium sulfate, concentrated under reduced pressure, and separated by flash preparative chromatography (dichloromethane/methanol, 15/1) under medium pressure to obtain Compound 3b (300 mg,37.44%) as an off-white solid. 1H NMR (400 MHz, DMSO-d6): δ ppm: 8.15 (s, 1H), 7.73 (d, J = 5.2 Hz, 1H), 6.96 (d, J = 5.6 Hz, 1H), 6.06 (s, 2H), 3.63 (s, 3H). LCMS: 209.0 [M+H]+.

### STEP B

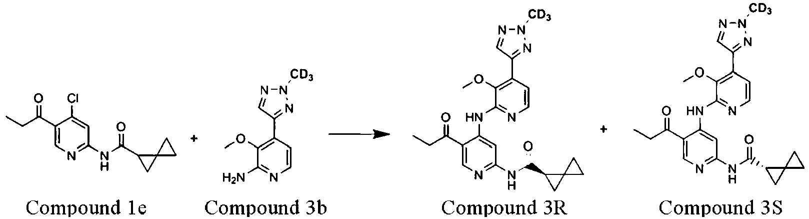

Compound 1e (65 mg, 0.23 mmol) and Compound 3b (48.5 mg, 0.23 mmol) were dissolved in dioxane (5 mL). The solution was successively added with XantPhos (53.9 mg, 0.093 mmol), cesium carbonate (227.9 mg, 0.70 mmol) and Pd2(dba)3 (42.7 mg, 0.047 mmol). The mixture was stirred at 115°C under microwave for 2 hours under nitrogen protection. The reaction was monitored by LCMS. After the completion of the reaction, the reaction was diluted with water (20 mL) and extracted with ethyl acetate (20 mL x 3). The extractions were combined, washed with saturated saline (20 mL), dried over anhydrous sodium sulfate and concentrated under reduced pressure. The residue was separated by flash preparative chromatography (dichloromethane/methanol, 20/1) under medium pressure to obtain 120 mg crude product. The crude product was separated by preparative HPLC (acetonitrile/water = 55:45; Gemini 5u C18 150 x 21.2 mm) to give 30 mg white solid, which was then separated by SFC (Thar Preparative 80, Column: CHIRALPAK IC 250 mm x 20 mm x 5 µm, Modifier: 40% methanol (NH4OH 0.2%)/60% CO2, Total flow rate: 40 g/min, Temperature: 40 °C) to give two isomers: Compound 3R (11.5 mg, Rt: 3.58 min), Compound 3S (12.6 mg, Rt: 7.23 min), Total yield: 22.93%.

Compound 3R: 1H NMR (400 MHz, DMSO) δ 12.32 (s, 1H), 10.72 (s, 1H), 9.68 (s, 1H), 8.95 (s, 1H), 8.32 (s, 1H), 8.17 (d, J = 5.2 Hz, 1H), 7.47 (d, J = 5.2 Hz, 1H), 3.81 (s,3H), 3.16 (q, J = 7.2 Hz, 2H), 2.42 (dd, J = 7.2, 4.3 Hz, 1H), 1.44 (t, J = 3.6 Hz, 1H), 1.35 (dd, J = 7.3, 3.1 Hz, 1H), 1.14 (t, J = 7.1 Hz, 3H), 0.97-0.82 (m, 3H),0.78 (d, J = 5.6 Hz, 1H). LCMS: 451.1 [M+H]+.

Compound 3S: 1H NMR (400 MHz, DMSO) δ 12.32 (s, 1H), 10.72 (s, 1H), 9.68 (s, 1H), 8.95 (s, 1H), 8.32 (s, 1H), 8.17 (d, J = 5.2 Hz, 1H), 7.47 (d, J = 5.2 Hz, 1H), 3.81 (s,3H), 3.16 (q, J = 7.2 Hz, 2H), 2.42 (dd, J = 7.2, 4.3 Hz, 1H), 1.44 (t, J = 3.6 Hz, 1H), 1.35 (dd, J = 7.3, 3.1 Hz, 1H), 1.14 (t, J = 7.1 Hz, 3H), 0.97-0.82 (m, 3H),0.78 (d, J = 5.6 Hz, 1H). LCMS: 451.2 [M+H]+.

### Example 4

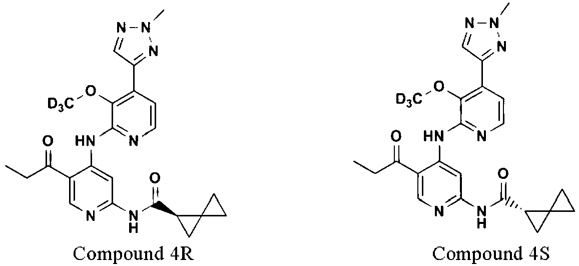

### STEP A

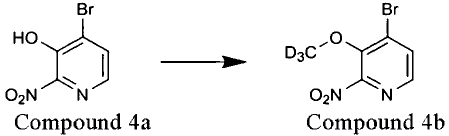

Compound 4a (10 g, 45.66 mmol), and potassium carbonate (12.62 g, 91.33 mmol) were successively added to a 250mL round bottom flask containing 100 mL N,N-dimethylformamide. The mixture was stirred at room temperature for 10 min and added with deuterated iodomethane (12.96 g, 91.33 mmol) and stirred at 30 °C for 20 hours. The reaction was monitored by LCMS. After the completion of the reaction, the reaction was filtered under reduced pressure, and the filtrate was concentrated under reduced pressure. The residue was added with 300 mL of water, and extracted with ethyl acetate (200 mL x 3). The extractions were combined, washed with saturated saline (50 mL), dried over anhydrous sodium sulfate, concentrated under reduced pressure, and separated by flash preparative chromatography (dichloromethane/methanol, 15/1) under medium pressure to obtain Compound 4b (5 g, 46.97%) as a yellow solid. LCMS: 236.0, 238.0 [M+H]+.

### STEP B

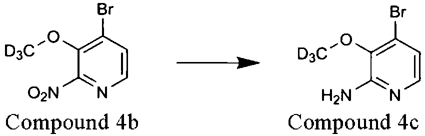

Compound 4b (5 g, 22.0 mmol), iron powder (4.92 g, 88.1 mmol), and ammonium chloride (4.71 g, 88.1 mmol) were successively added to a 500mL round bottom flask containing 90 mL of ethanol and 30 mL of water. The mixture was stirred at 70 °C for 3 hours. The reaction was monitored by LCMS. After the completion of the reaction, the reaction was filtered, and the filtrate was concentrated under reduced pressure. The residue was diluted by adding 50 mL of water, and extracted with ethyl acetate (200 mL x 3). The extractions were combined, dried over anhydrous Na2SO4, and concentrated under reduced pressure, and separated by column chromatography (petroleum ether/ethyl acetate, 1/1) to obtain Compound 4c (3.3 g,72.7%) as a yellow solid. 1H NMR (400 MHz, CDCl3): δ ppm: 7.60 (d, J = 5.3 Hz, 1H), 6.81 (d, J = 5.2 Hz, 1H), 4.82 (s, 2H).LCMS: 206.0, 208.0[M+H]+.

### STEP C

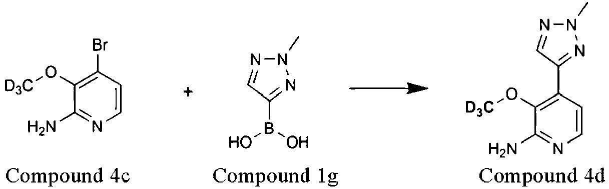

Compound 4c (1.0 g, 4.85 mmol) and Compound 1g (650 mg, 5.34 mmol) were dissolved in a mixed solution of dioxane (20 mL) and water (4 mL). The solution was sequentially added with potassium carbonate (2.02 g, 14.55 mmol) and Pd(dppf)Cl2-CH2C12 (400.1 mg, 0.49 mmol), and the mixture was stirred for 16 h at 110 °C under nitrogen protection. The reaction was monitored by LCMS. After the completion of the reaction, the reaction was filtered, and the filtrate was concentrated under reduced pressure. The residue was diluted by adding water (100 mL), and extracted with ethyl acetate (150 mL x 3). The extractions were combined, washed with saturated saline (50 mL), dried over anhydrous sodium sulfate, concentrated under reduced pressure, and separated by flash preparative chromatography (petroleum ether/ethyl acetate, 1/2) under medium pressure to obtain Compound 4d (300 mg,29.7%) as a yellow solid. 1H NMR (400 MHz, CDCl3): δ ppm 8.10 (s, 1H), 7.87 (d, J = 5.2 Hz, 1H), 7.18 (d, J = 5.2 Hz, 1H), 4.84 (s, 2H), 4.28 (s, 3H). LCMS: 209.2 [M+H]+.

### STEP D

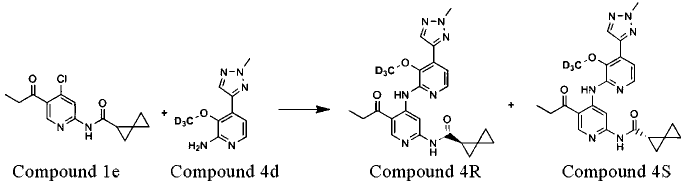

Compound 1e (65 mg, 0.23 mmol) and Compound 4d (48.5 mg, 0.23 mmol) were dissolved in dioxane (5 mL). The solution was successively added with XantPhos (53.9 mg, 0.093 mmol), cesium carbonate (227.9 mg, 0.70 mmol) and Pd2(dba)3 (42.7 mg, 0.047 mmol). The mixture was stirred at 115°C under microwave for 2 hours under nitrogen protection. The reaction was monitored by LCMS. After the completion of the reaction, the reaction was diluted with water (20 mL) and extracted with ethyl acetate (20 mL x 3). The extractions were combined, washed with saturated saline (20 mL), dried over anhydrous sodium sulfate and concentrated under reduced pressure. The residue was separated by flash preparative chromatography (dichloromethane/methanol, 20/1) under medium pressure to obtain 200 mg crude product. The crude product was separated by preparative HPLC (acetonitrile/water = 55:45; Gemini 5u C18 150 x 21.2 mm) to give 30 mg product, which was then separated by SFC (Thar Preparative 80, Column: CHIRALPAK IC 250 mm x 20 mm x 5 µm, Modifier: 40% methanol (NH4OH 0.2%)/60% CO2, Total flow rate: 40 g/min, Temperature: 40 °C) to give two isomers: Compound 4R (10.3 mg, Rt: 3.54 min), Compound 4S (9.2 mg, Rt: 7.07 min), Total yield: 18.53%.

Compound 4R: 1H NMR (400 MHz, DMSO) δ 12.33 (s, 1H), 10.73 (s, 1H), 9.69 (s, 1H), 8.95 (s, 1H), 8.33 (s, 1H), 8.18 (d, J = 5.2 Hz, 1H), 7.47 (d, J = 5.2 Hz, 1H), 4.28 (s,3H), 3.17 (q, J = 7.1 Hz, 2H), 2.42 (dd, J = 7.2, 4.4 Hz, 1H), 1.44 (t, J = 3.7 Hz, 1H), 1.36 (dd, J = 7.4, 3.3 Hz, 1H), 1.14 (t, J = 7.1 Hz, 3H), 0.95- 0.82 (m, 3H),0.78 (d, J = 5.5 Hz, 1H). LCMS: 451.1 [M+H]+.

Compound 4S:1H NMR (400 MHz, DMSO) δ 12.33 (s, 1H), 10.73 (s, 1H), 9.69 (s, 1H), 8.95 (s, 1H), 8.33 (s, 1H), 8.18 (d, J = 5.2 Hz, 1H), 7.47 (d, J = 5.2 Hz, 1H), 4.28 (s,3H), 3.17 (q, J = 7.1 Hz, 2H), 2.42 (dd, J = 7.2, 4.4 Hz, 1H), 1.44 (t, J = 3.7 Hz, 1H), 1.36 (dd, J = 7.4, 3.3 Hz, 1H), 1.14 (t, J = 7.1 Hz, 3H), 0.95 - 0.82 (m, 3H),0.78 (d, J = 5.5 Hz, 1H).LCMS: 451.1 [M+H]+.

### Example 5

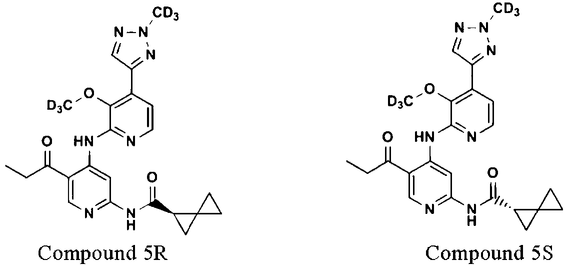

### STEP A

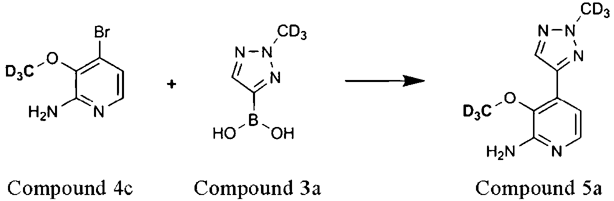

Compound 4c (793.3 mg, 3.85 mmol) and Compound 3a (500 mg, 3.85 mmol) were dissolved in a mixed solution of dioxane (20 mL) and water (4 mL). The solution was sequentially added with potassium carbonate (1.6 g, 11.55 mmol) and (318.5 mg, 0.39 mmol), and the mixture was stirred for 16 h at 90 °C under nitrogen protection. The reaction was monitored by LCMS. After the completion of the reaction, the reaction was filtered, and the filtrate was concentrated under reduced pressure. The residue was diluted by adding water (100 mL), and extracted with ethyl acetate (150 mL x 3). The extractions were combined, washed with saturated saline (50 mL), dried over anhydrous sodium sulfate, concentrated under reduced pressure, and separated by flash preparative chromatography (petroleum ether/ethyl acetate, 1/2) under medium pressure to obtain Compound 5a (400 mg, 49.1%) as a yellow solid.

1H NMR (400 MHz, CDCl3): δ ppm 8.11 (s, 1H), 7.86 (s, 1H), 7.18 (d, J = 5.2 Hz, 1H), 4.86 (s, 2H). LCMS: 212.3 [M+H]+.

### STEP B

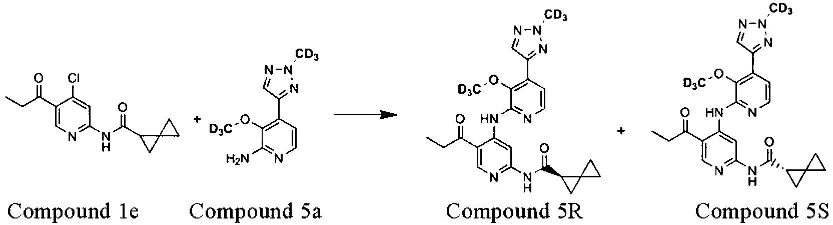

Compound 1e (65 mg, 0.23 mmol) and Compound 5a (48.5 mg, 0.23 mmol) were dissolved in dioxane (5 mL). The solution was successively added with XantPhos (53.9 mg, 0.093 mmol), cesium carbonate (227.9 mg, 0.70 mmol) and Pd2(dba)3 (42.7 mg, 0.047 mmol). The mixture was stirred at 115 °C under microwave for 2 hours under nitrogen protection. The reaction was monitored by LCMS. After the completion of the reaction, the reaction was diluted with water (20 mL) and extracted with ethyl acetate (20 mL x 3). The extractions were combined, washed with saturated saline (20 mL), dried over anhydrous sodium sulfate and concentrated under reduced pressure. The residue was separated by flash preparative chromatography (dichloromethane/methanol, 20/1) under medium pressure to obtain 200mg crude product. The crude product was separated by preparative HPLC (acetonitrile/water = 55:45; Gemini 5u C18 150 x 21.2 mm) to give 25 mg of product, which was then separated by SFC (Thar Preparative 80, Column: CHIRALPAK IC 250 mm x 20 mm x 5 µm, Modifier: 40% methanol (NH4OH 0.2%)/60% CO2, Total flow rate: 40 g/min, Temperature: 40 °C) to give two isomers: Compound 5R (4.3 mg, Rt: 3.78 min), Compound 5S (6.4 mg, Rt: 7.67 min), Total yield: 10.14%.

Compound 5R: 1H NMR (400 MHz, DMSO) δ 12.33 (s, 1H), 10.74 (s, 1H), 9.69 (s, 1H), 8.95 (s, 1H), 8.33 (s, 1H), 8.17 (d, J = 5.2 Hz, 1H), 7.47 (d, J = 5.2 Hz, 1H), 3.17 (q, J= 7.1 Hz, 2H), 2.42 (dd, J = 7.2, 4.5 Hz, 1H), 1.44 (t, J = 3.7 Hz, 1H), 1.39 - 1.33 (m, 1H), 1.14 (t, J = 7.1 Hz, 3H), 0.97-0.82 (m, 3H), 0.78 (d, J = 5.4 Hz, 1H). LCMS: 454.2 [M+H]+.

Compound 5S: 1H NMR (400 MHz, DMSO) δ 12.33 (s, 1H), 10.74 (s, 1H), 9.69 (s, 1H), 8.95 (s, 1H), 8.33 (s, 1H), 8.17 (d, J = 5.2 Hz, 1H), 7.47 (d, J = 5.2 Hz, 1H), 3.17 (q, J= 7.1 Hz, 2H), 2.42 (dd, J = 7.2, 4.5 Hz, 1H), 1.44 (t, J = 3.7 Hz, 1H), 1.39 - 1.33 (m, 1H), 1.14 (t, J = 7.1 Hz, 3H), 0.97-0.82 (m, 3H), 0.78 (d, J = 5.4 Hz, 1H). LCMS: 454.0 [M+H]+.

### Example 6

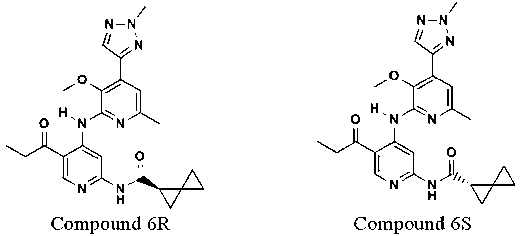

### STEP A

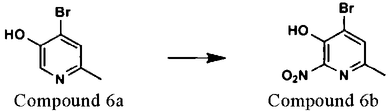

Compound 6a (300 mg, 1.59 mmol) was dissolved in concentrated sulfuric acid (4 mL) at 0°C, fuming nitric acid (2 mL) was added dropwise, and the mixture was stirred at 0°C for 1 hour. The reaction was monitored by LCMS. After the completion of the reaction, the reaction solution was added into ice water (10 mL) and stirred for 5 min, then extracted with ethyl acetate (50 mL x 2). The extractions were combined, washed with saturated sodium bicarbonate aqueous solution (50 mL) and saturated saline (50 mL) respectively, dried over anhydrous sodium sulfate, concentrated under reduced pressure, and separated by flash preparative chromatography (dichloromethane/methanol, 20/1) under medium pressure to obtain Compound 6b (140 mg, 28.24%) as a yellow solid. 1H NMR (400 MHz, DMSO) δ 11.25 (s, 1H), 7.98 (s, 1H), 2.41(s, 3H). LCMS: Rt = 1.14 min, MS 233.1 [M+H] +.

### STEP B

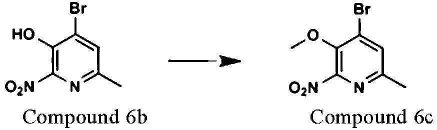

Compound 6b (140 mg, 0.60 mmol) was dissolved in N,N-dimethylformamide (5 mL), potassium carbonate (166.0 mg, 1.20 mmol) was added and the mixture was stirred for 0.5 h at room temperature. Iodomethane (170.5 mg, 1.20 mmol) was added dropwise under nitrogen protection, and the mixture was continuously stirred for 16 h at room temperature. The reaction was monitored by LCMS. After the completion of the reaction, the reaction was diluted by adding water (50 mL) and extracted with ethyl acetate (50 mL x 2). The extractions were combined, washed with saturated saline (50 mL), dried over anhydrous sodium sulfate, concentrated under reduced pressure, and separated by flash preparative chromatography (acetate ethyl/petroleum ether, 1/10) under medium pressure to obtain Compound 6c (144 mg, 97.02%) as an off-white solid. LCMS: 247.0 [M+H]+.

### STEP C

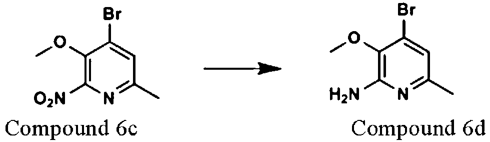

Compound 6c (144 mg, 0.58 mmol) was dissolved in a mixed solvent containing ethanol:acetic acid:water (5 mL:5 mL:2.5 mL), iron powder (180.8 mg, 3.23 mmol) was added at room temperature, and the reaction was stirred under nitrogen protection for 2 hours at room temperature. The reaction was monitored by LCMS. After the completion of the reaction, the reaction was filtered under reduced pressure, and the filtrate was concentrated under reduced pressure. The residue was added with 30 mL of water, and extracted with ethyl acetate (50 mL x 2). The extractions were combined, washed with saturated saline (50 mL), dried over anhydrous sodium sulfate, concentrated under reduced pressure, and separated by flash preparative chromatography (dichloromethane/methanol, 20/1) under medium pressure to obtain Compound 6d (120 mg, 94.81%) as a yellow solid. LCMS: MS 217.1 [M+H]+ .

### STEP D

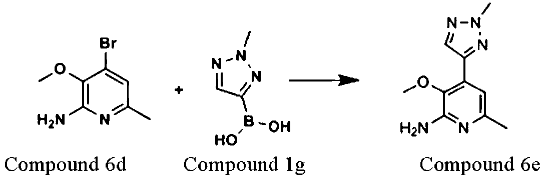

Compound 6d (120 mg, 0.5527 mmol) and Compound 1g (122.79 mg, 0.96 mmol) were dissolved in a mixed solution of dioxane (10 mL) and water (1 mL). The solution was sequentially added with potassium carbonate (267.44 mg, 1.93 mmol) and [1,1'-bis(diphenylphosphino)ferrocene]palladium(II) dichloride dichloromethane complex (52.67 mg, 0.06 mmol), and the mixture was stirred for 16 h at 110 °C under nitrogen protection. The reaction was monitored by LCMS. After the completion of the reaction, the reaction was filtered, and the filtrate was concentrated under reduced pressure. The residue was diluted with water (100 mL), and extracted with ethyl acetate (50 mL x 2). The extractions were combined, washed with saturated saline (50 mL), dried over anhydrous sodium sulfate, concentrated under reduced pressure, and separated by flash preparative chromatography (dichloromethane/methanol, 20/1) under medium pressure to obtain Compound 6e (110 mg, 90.79%) as a yellow solid. 1H NMR (400 MHz, DMSO) δ 8.13 (s, 1H), 6.83 (s, 1H), 5.98 (s, 2H), 4.22 (s, 3H), 3.60 (s, 3H), 2.24 (s, 3H). LCMS: MS 220.2 [M+H] +.

### STEP E

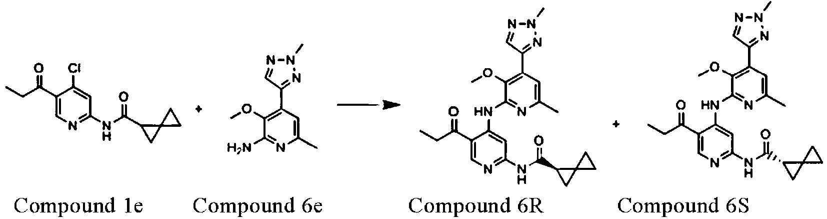

Compound 1e (33 mg, 0.12 mmol) and Compound 6e (25.9 mg, 0.12 mmol) were dissolved in dioxane (5 mL). The solution was successively added with XantPhos (27.4 mg, 0.047 mmol), cesium carbonate (115.7 mg, 0.36 mmol) and Pd2(dba)3 (21.6 mg, 0.024 mmol). The mixture was stirred at 115°C under microwave for 2 hours under nitrogen protection. The reaction was monitored by LCMS. After the completion of the reaction, the reaction was diluted with water (20 mL) and extracted with ethyl acetate (20 mL x 3). The extractions were combined, washed with saturated saline (20 mL), dried over anhydrous sodium sulfate and concentrated under reduced pressure. The residue was separated by flash preparative chromatography (dichloromethane/methanol, 20/1) under medium pressure to obtain 50 mg crude product. The crude product was separated by preparative HPLC (acetonitrile/water = 55:45; Gemini 5u C18 150 x 21.2 mm) to give 20 mg of racemic product, which was then separated by SFC (Thar Preparative 80, Column: CHIRALPAK IC 250 mm x 20 mm x 5 µm, Modifier: 40% methanol (NH4OH 0.2%)/60% CO2, Total flow rate: 40 g/min, Temperature: 40 °C) to give two isomers: Compound 6R (3.7 mg, Rt: 3.65 min), Compound 6S (2.5 mg, Rt: 7.82 min), Total yield: 11.2%.

Compound 6R : 1H NMR (400 MHz, DMSO) δ 12.25 (s, 1H), 10.70 (s, 1H), 9.92 (s, 1H), 8.94 (s, 1H), 8.29 (s, 1H), 7.33 (s, 1H), 4.27 (s, 3H), 3.78 (s, 3H), 3.16 (q, J = 7.2 Hz,2H), 2.51 (s, 3H), 2.42 (dd, J = 7.3, 4.4 Hz, 1H), 1.43 (t, J = 3.8 Hz, 1H), 1.35 (dd, J = 7.4, 3.3 Hz, 1H), 1.13 (t, J = 7.2 Hz, 3H), 0.88 (dt, J = 8.4, 4.8 Hz, 3H),0.77 (d, J = 5.5 Hz, 1H). LCMS: 462.0 [M+H]+.

Compound 6S : 1H NMR (400 MHz, DMSO) δ 12.25 (s, 1H), 10.70 (s, 1H), 9.92 (s, 1H), 8.94 (s, 1H), 8.29 (s, 1H), 7.33 (s, 1H), 4.27 (s, 3H), 3.78 (s, 3H), 3.16 (q, J = 7.2 Hz,2H), 2.51 (s, 3H), 2.42 (dd, J = 7.3, 4.4 Hz, 1H), 1.43 (t, J = 3.8 Hz, 1H), 1.35 (dd, J = 7.4, 3.3 Hz, 1H), 1.13 (t, J = 7.2 Hz, 3H), 0.88 (dt, J = 8.4, 4.8 Hz, 3H),0.77 (d, J = 5.5 Hz, 1H). LCMS: 462.0 [M+H]+.

### Example 7

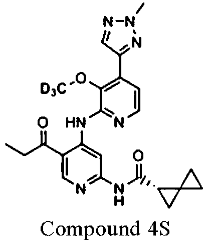

### STEP A

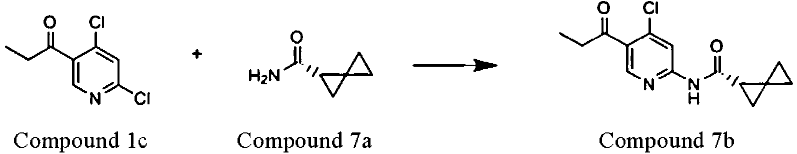

Compound 1c (450 mg, 2.22 mmol) and Compound 7a (370 mg, 3.33 mmol) were dissolved in dioxane (15 mL). The solution was successively added with Pd2(dba)3 (360 mg, 0.44 mmol), cesium carbonate (1.44 g, 4.41 mmol) and 1, 1'-bis(dicyclohexylphosphino)ferrocene (dcpf, 335.5 mg, 0.59 mmol). The mixture was stirred at 80 °C for 3 hours under nitrogen protection. The reaction was monitored by LCMS. After the completion of the reaction, the reaction was concentrated under reduced pressure, diluted by adding water (20 mL), and extracted with ethyl acetate (20 mL x 3). The extractions were combined, washed with saturated saline (20 mL), dried over anhydrous sodium sulfate, and concentrated under reduced pressure. The residue was separated by column chromatograph (petroleum ether/ethyl acetate, 3/1) to obtain Compound 7b (521 mg, 84.4%) as a yellow solid. 1H NMR (400 MHz, CD3OD) δ 8.59 (s, 1H), 8.33 (s, 1H), 3.04 (q, J = 7.2 Hz, 2H), 2.27 (dd, J = 7.4, 4.2 Hz, 1H), 1.58 (t, J = 3.9 Hz, 1H), 1.47 (dd, J = 7.4, 3.6 Hz, 1H), 1.19 (t, J = 7.2 Hz, 3H), 1.04-0.94 (m, 3H), 0.92-0.87 (m, 1H). LCMS: 279.1, 281.1[M+H]+.

### STEP B

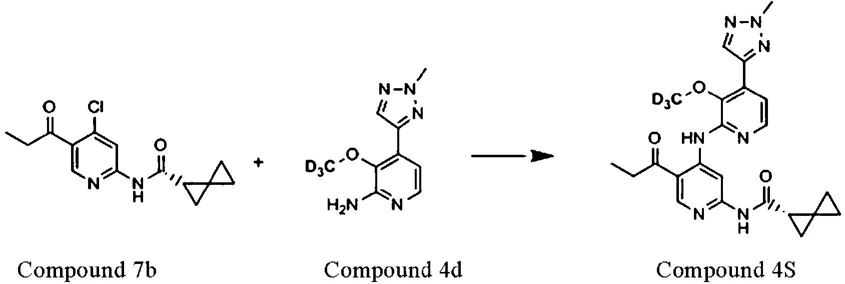

Compound 7b (65 mg, 0.23 mmol) and Compound 4d (48.54 mg, 0.23 mmol) were dissolved in dioxane (5 mL). The solution was successively added with XantPhos (54 mg, 0.093 mmol), cesium carbonate (228 mg, 0.70 mmol) and Pd2(dba)3 (42.7 mg, 0.047 mmol). The mixture was stirred at 115°C under microwave for 2 hours under nitrogen protection. The reaction was monitored by LCMS. After the completion of the reaction, the reaction was diluted with water (20 mL) and extracted with ethyl acetate (20 mL x 3). The extractions were combined, washed with saturated saline (20 mL), dried over anhydrous sodium sulfate and concentrated under reduced pressure. The residue was separated by flash preparative chromatography (dichloromethane/methanol, 20/1) under medium pressure to obtain 150 mg crude product. The crude product was isolated by preparative HPLC (acetonitrile/water, 55/45; Gemini 5u C18 150 x 21.2 mm) to give compound 4S (30 mg, 29%) as a white solid (OR: 110, 23.2 °C). 1H NMR (400 MHz, DMSO) δ 12.33 (s, 1H), 10.73 (s, 1H), 9.69 (s, 1H), 8.95 (s, 1H), 8.33 (s, 1H), 8.17 (d, J = 5.2 Hz, 1H), 7.47 (d, J = 5.2 Hz, 1H), 4.28 (s, 3H), 3.17 (q, J = 7.2 Hz, 2H), 2.43 (dd, J = 7.4, 4.3 Hz, 1H), 1.44 (t, J = 3.8 Hz, 1H), 1.36 (dd, J = 7.4, 3.3 Hz, 1H), 1.14 (t, J = 7.2 Hz, 3H), 0.98 - 0.83 (m, 3H), 0.78 (dd, J = 8.2, 2.7 Hz, 1H). LCMS: 451.1 [M+H]+.

### Test Example

### Test Example 1: The inhibitory effect of compounds on the expression of pSTAT5 in CD3+cells using FACS assay

### 1. Experimental Materials and instruments:

### 1.1. Experimental reagents

- DMSO, Sigma, Cat# D2650-100 mL, stored at room temperature.
- Perm buffer III, BD Biosciences, Cat # 558050, stored at 4 °C.
- Lyse/Fix buffer, BD Biosciences, Cat # 558049, stored at room temperature.
- EDTA, Invitrogen, Cat # 15575-038, stored at room temperature.
- PBS, Hyclone, Cat # SH30256.01, stored at 4 °C.
- PE Mouse Anti-Human CD3, BD Biosciences, Cat # 555333, stored at 4 °C.
- Mouse anti-human Phospho-STATS (pY694) (Alexa Fluor^{®} 647 Conjugate), BD Biosciences, Cat# 562076, stored at 4 ° C.
- IFN- α, Biolegend, Cat # 592702.

### 1.2 Experimental consumables

- Microplate, 96 Well, PP, v-bottom, Greiner, Cat # GN651201-100EA.
- 5 mL polystyrene round bottom tube, FALCON, Cat # 04318011.
- 96 square well storage plate, Thermo, Cat # AB-0661.
- 96-well plate, Corning, Cat # 3599.

### 1.3 Instruments

- CO2 cell incubator: MCO-15AC Biosciences (Thermo).
- Pipette: 0.2-10 µL, 20-200 µL, 200-1000 µL (Thermo).
- Multichannel pipette: 0.2-10 µL, 5-50 µL, 20-300 µL (Raining).
- Centrifuge: Thermo Centrifuge ST 40R; Thermo LEGEND Micro 21R.
- Water system: Millipore Milli-Q Reference system.
- Freezer: Haier ultralow temperature freezer.
- Haier 4 degree refrigerator.
- Haier -20 degree freezer.
- Vortex: EARTH REQUIRED.
- Plate Shaker: QI LIN BEI ER; MH-2.
- Flow Cytometer: BD FACSVerseTM Flow Cytometer.

### 2 Experimental methods:

### 2.1 Compound Dilution

1) On the day of experiment, the compound was prepared into a 10mM solution with DMSO, diluted to 1.5mM in DMSO, and then diluted to 8 gradient concentrations in 3-fold dilutions Concentrations.
2) 5 µL of the diluted compound was transferred into 120ul of DPBS solution containing 0.1% BSA.
3) Positive and negative control groups were set up, and 0.2% DMSO was added to the positive and negative control groups.

### 2.2 Experimental procedure

1) 0.5 million and 67.5ul of human PBMC cells was added to the 96-well cell culture plate per well.
2) 3.5ul of diluted compound was added and mixed well.
3) Incubation was conducted in a 37 ° C incubator for 60 minutes.
4) IFN- alpha was diluted in DPBS containing 0.1% BSA to 600ng/mL, and PE anti-hCD3 antibody was diluted in DPBS containing 0.1% BSA to 3 fold. When the above 60-minute incubation was complete, diluted PE anti-hCD3 antibody was added at 5ul per well and diluted IFN-α was added at 4ul per well, i.e., the final concentration of IFN-α per well is 20ng/ml.
5) Incubation was conducted in a 37 ° C incubator for 30 minutes.
6) All cells were transferred to a 96-well deep well plate and 1ml of 37 ° C preheated lysis/fix buffer was added.
7) Incubation was conducted at 37 ° C in dark for 10 minutes.
8) Centrifugation was carried out at 600g for 5 minutes and then the supernatant was discarded. 1ml PBS was added to wash for twice and then centrifuged.
9) 1ml of Perm buffer III was added to the cell precipitation.
10) Incubation was conducted at 4° C in dark for 30 minutes.
11) Centrifugation was carried out at 600g for 5 minutes and then the supernatant was discarded. 1ml PBS was added to wash for twice and then centrifuged.
12) APC anti-human pSTAT5 antibody was diluted in staining buffer by 200-fold, which was added to cell wells at 100uL per well and mixed well.
13) Incubation was conducted at room temperature for 40 minutes.
14) Staining buffer was added to wash for twice with 1mL for each well, and centrifugation was carried out at 600g for 5 minutes.
15) After discarding the supernatant, the cell precipitation was re-suspended in 300uL of staining buffer.
16) Sample loading and analysis were carried out in a flow cytometer. The IC50 of the samples to be tested was obtained from the above, Table 1.

**Table 1**

| Compound No. | IFN-alpha/pSTAT5 IC50 (nM) |
|---|---|
| 1S | A |
| 1R | B |
| 2S | B |
| 2R | B |
| 3S | A |
| 3R | A |
| 4S | A |
| 4R | B |
| 5S | A |
| 5R | B |
| 6S | A |
| 6R | A |

| | |
|---|---|
| A < 50nM, B > 50nM. | |

### Test Example 2: Comparison of selectivity of the JAK family

### 1. Experimental method

1.1. Pseudo kinase experiment operation was as follows:
1.1.1. The compound was dissolved with DMSO to a storage concentration of 10mM.
1.1.2. A compound concentration of 200 times the final concentration was prepared in a compound dilution plate, which was diluted from the highest concentration point with a total of 4 concentration points using the 27 times dilution method, and transferred to the Echo plate.
1.1.3. The compound was flushed from the Echo plate to the 384-well experimental plate using an Echo instrument, and the compound become 11 concentration points of the 3-fold dilution matrix.
1.1.4. 5 µl of 3X TYK2(JH2) or JAK1(JH2) kinase was added to the 384-well experimental plate.
1.1.5. 5 µl of 3X Tb was added to the 384-well experimental plate.
1.1.6.5 µl of 3X Tracer was added to the 384-well experimental plate.
1.1.7. Centrifugation was conducted for 30 seconds and incubation was carried out at room temperature for 60 minutes.
1.1.8. The signal values were read using Envision Microplate Reader (PerkinElmer).
1.2. Kinase experiment operation was as follows:
1.2.1. The compound was dissolved with DMSO to a storage concentration of 10mM.
1.2.2. A compound concentration of 100 times the final concentration was prepared in a compound dilution plate, which was diluted from the highest concentration point with a total of 4 concentration points using the 27 times dilution method, and transferred to the Echo plate.
1.2.3. The compound was flushed from the Echo plate to the 384 experimental plate using an Echo instrument, and the compound become 11 concentration points of the 3-fold dilution matrix.
1.2.4. 2X kinase working solution was prepared, which was added to a 384-well experimental plate at 5 µl per well. The compound and the kinase were incubated at room temperature for 15 minutes.
1.2.5. 5ul of 2X substrate (containing ATP) was added to the 384 well plate.
1.2.6. Incubation was conducted at room temperature for 45 minutes.
1.2.7. The detection reagent mixture was added to the 384-well plate, and centrifuged for 30 seconds, and incubation was conducted at room temperature for 60 minutes.
1.2.8. The signal values were read using Envision Microplate Reader (PerkinElmer).

### 1.3. Data Analysis

1.3.1. XL-Fit software was used for data analysis to obtain IC50 of the compounds, Table 2.

**Table 2**

| Compound No. | TYK2 (JH1) (nM) | TYK2 (JH2) (nM) | JAK1 (JH1) (nM) | JAK1 (JH2) (nM) | JAK2 (JH1) (nM) | JAK3 (JH1) (nM) |
|---|---|---|---|---|---|---|
| 4S | >9900 | 0.29 | >9900 | 21.99 | 5419 | >9900 |

### Test Example 3: Research on Animal Model of Multiple Sclerosis

Experimental Autoimmune Encephalomyelitis (EAE) is a demyelinating disease of the central nervous system and is a common animal model for multiple sclerosis. The aim of this experiment was to investigate the efficacy of compound 4S in terms of clinical disease and histopathology in myelin oligodendrocyte glycoprotein (MOG)-induced EAE in mice.

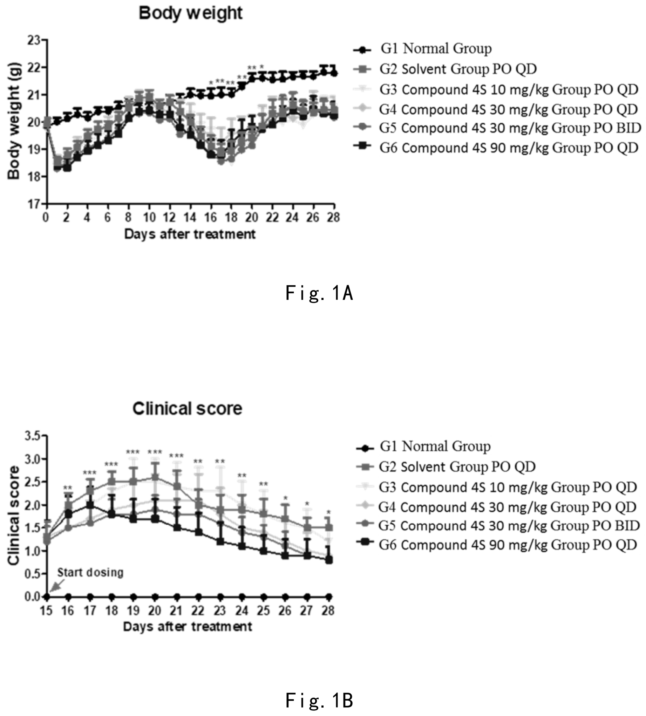

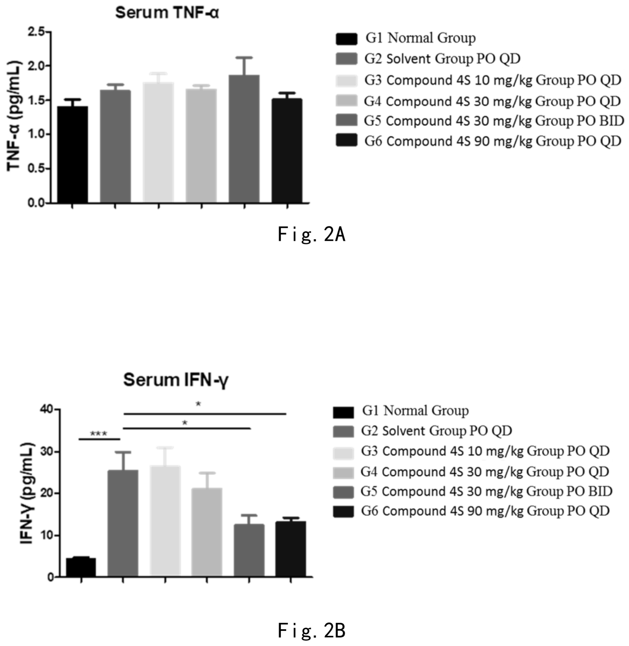

60 female C57BL/6 mice of Specific pathogen free (SPF) grade were randomly divided into six groups according to body weight: normal control group, model (solvent) group, compound 4S low dose (10 mg/kg, QD) group, compound 4S medium dose (30 mg/kg, QD) group, compound 4S medium dose (30 mg /kg, BID) group and Compound 4S high dose (90 mg/kg, QD) group with 10 animals in each group. Except the normal group, mice in the remaining groups were each injected subcutaneously with 200 µL of MOG 35-55 emulsion to induce EAE model. Immunization was reinforced by intraperitoneal injection of 250 µL of pertussis toxin (PTX, 1 µg/mL) per mouse at 0 and 48 h after MOG immunization. Except the normal group, mice in the remaining groups were given solvent (ethanol: Vitamin E polyethylene glycol succinate: polyethylene glycol 300 = 5:5:90) or Compound 4S daily starting on the 15th day after immunization (Day 15) and continuing until Day 28 for a total of 14 days. The health status of the animals was closely monitored during the experiments, and their body weights and clinical scores were recorded daily (Figs. 1A, 1B). On Day 28, after euthanizing mice by carbon dioxide, blood samples were collected and the levels of TNF-α and IFN-γ in serum were measured by ELISA (Figure 2A, 2B).

### Test Example 4: Analysis of Drug Concentrations in Plasma Samples and Brain Tissue Samples

The purpose of the study was to evaluate drug concentrations in plasma samples and brain tissue samples of female C57BL/6 mice after 15 consecutive days of oral administration of 10 mg/kg, 30 mg/kg, and 90 mg/kg of Compound 4S. The drug concentrations of Compound 4S in plasma samples and brain tissue homogenate samples were detected by the positive ion (ESI) multi-reactive ion monitoring (MRM) scanning mode of LC-MS instrument with a linear range of 1- 10000 ng/mL.

Plasma samples from 10 mice orally administered with Compound 4S: For standard curves, QC samples, single blanks, and unknown samples, the sample volume was 10 µL and each was added with 200 µL of methanol: acetonitrile = 1:1 (v/v) precipitant (containing 5 ng/mL of Terfenadine). For double-blank samples, each was added with 200 µL of Methanol: Acetonitrile = 1:1 (v/v) precipitant. The samples were vortexed for 1 min and centrifuged for 15 min (4000 rpm, 4°C) to obtain the supernatant solution, which was diluted by 10-fold using methanol: water=1:1 (v/v, containing 0.1% FA) as the diluent solution and then subjected to LC-MS/MS analysis.

Brain tissue homogenate samples from 10 mice orally administered with Compound 4S: For standard curves, QC samples, single blanks, and unknown samples, the sample volume was 50 µL and each was added with 200 µL of methanol: acetonitrile = 1:1 (v/v) precipitant (containing 5 ng/mL of Terfenadine). For double-blank samples, a 50 µL sample of blank brain tissue homogenate was taken and added with 200 µL of methanol/acetonitrile (1:1, v/v) precipitant. The samples were vortexed for 1 min and centrifuged for 15 min (4000 rpm, 4°C) to obtain the supernatant solution, which was diluted by 10-fold using methanol: water=1:1 (v/v, containing 0.1% FA) as the diluent solution and then subjected to LC-MS/MS analysis. Drug exposures in plasma samples and brain tissue samples at different administered doses and at different frequencies of administration of the same administered dose were compared (Table 3).

**Table 3**

| Dose | concentration in plasma (ng/ml) | concentration in brain (ng/ml) | Ratio |
|---|---|---|---|
| 10 mg QD | 784 | 615 | 0.788 |
| 30 mg QD | 2036 | 2355 | 1.11 |
| 30 mg BID | 2743 | 2359 | 0.88 |
| 90 mg QD | 4718 | 6261 | 1.32 |

2Hr after the last dose, by comparing the concentrations in plasma samples and brain tissue samples with different dosages, it was found that the concentration of the drug showed a significant increase with the elevation of the dosage, and there was a relatively obvious dose-dependence; comparing the same dosage with different dosage frequencies, it was found that the concentration did not increase with the increase of dosage frequency in plasma samples and brain tissue samples. In addition, the concentration ratio of brain tissue samples to plasma samples ranged from 0.788-1.32, indicating moderate brain permeability of the drug. By comparing the dose variability of the plasma concentrations, it showed that the plasma concentration ratio was slightly lower than the dose elevation, and the brain sample concentration ratio is slightly higher than the dose elevation.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A compound or a pharmaceutically acceptable salt thereof, wherein the compound is of formula A; 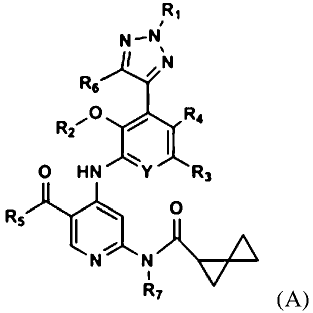 wherein,
Y is N or CR₉;
R₁ is selected from the group consisting of: H, and optionally substituted C₁₋₄alkyl;
R₂ is selected from the group consisting of: H, and optionally substituted C₁₋₄alkyl;
R₃ and R₄ are each independently selected from the group consisting of: H, halogen and optionally substituted C₁₋₄alkyl;
R₅ is selected from the group consisting of: H, and optionally substituted C₁₋₆alkyl;
R₆ is selected from the group consisting of: H, and optionally substituted C₁₋₄alkyl;
R₇ is selected from the group consisting of: H, and optionally substituted C₁₋₄alkyl;
R₉ is selected from the group consisting of: H, and optionally substituted C₁₋₄alkyl;
the "optionally substituted" means that the group is unsubstituted or is substituted with one or more substituents selected from the group consisting of: deuterium, halogen, C₁₋₄ alkyl, and C₁₋₄ haloalkyl.

2. The compound of claim 1 or the pharmaceutically acceptable salt thereof, wherein Y is N or CH.

3. The compound of claim 1 or the pharmaceutically acceptable salt thereof, wherein R₁ is selected from the group consisting of: C₁₋₄alkyl, and deuterated C₁₋₄alkyl.

4. The compound of claim 1 or the pharmaceutically acceptable salt thereof, wherein R₂ is selected from the group consisting of: C₁₋₄alkyl, and deuterated C₁₋₄alkyl.

5. The compound of claim 1 or the pharmaceutically acceptable salt thereof, wherein R₃ is selected from the group consisting of: H, and C₁₋₄alkyl.

6. The compound of claim 1 or the pharmaceutically acceptable salt thereof, wherein the compound is of formula I

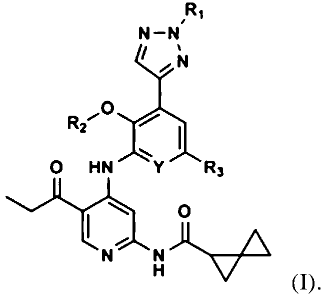

7. The compound of claim 1 or the pharmaceutically acceptable salt thereof, wherein the compound is of formula I-S or formula I-R

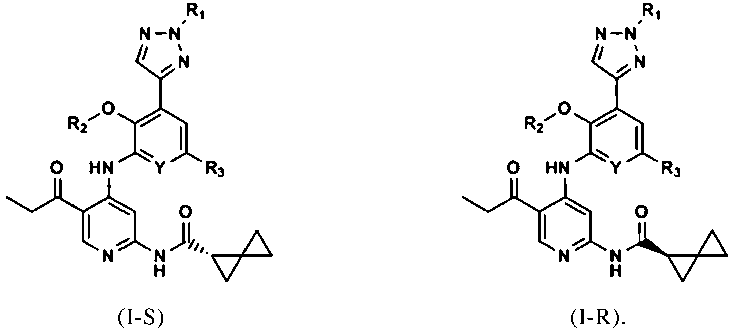

8. The compound of claim 1 or the pharmaceutically acceptable salt thereof, wherein the compound is a compound selected from Table A or Table B or a pharmaceutically acceptable salt thereof:
**Table A**
| | | | |
|---|---|---|---|
| 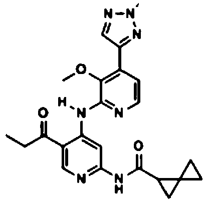 1 | 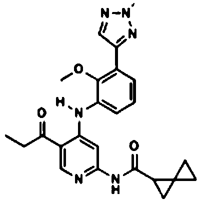 2 | 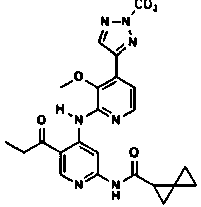 3 | |
| 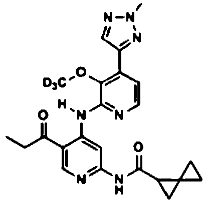 4 | 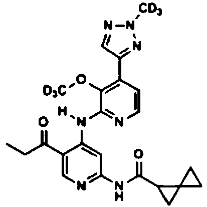 5 | 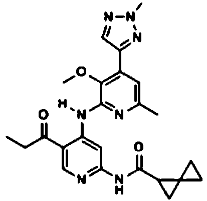 6 | |
**Table B**
| | | | |
|---|---|---|---|
| 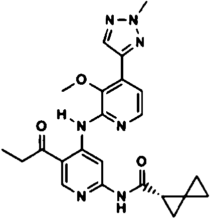 1S | 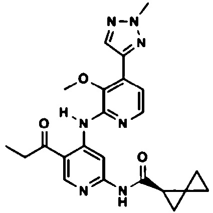 1R | 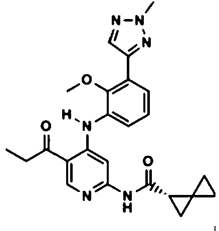 2S | 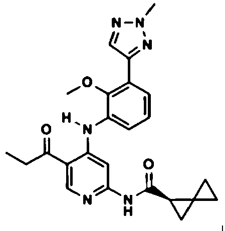 2R |
| 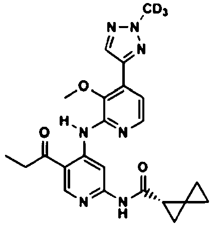 3S | 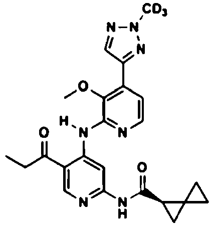 3R | 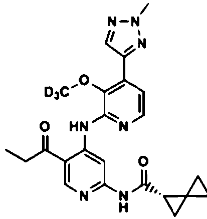 4S | 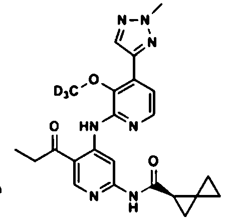 4R |
| 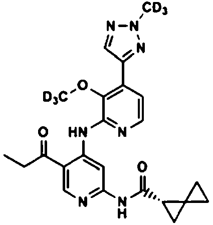 5S | 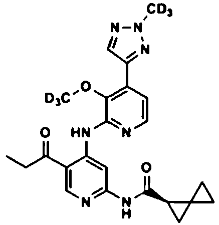 5R | 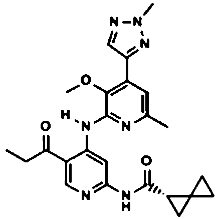 6S | 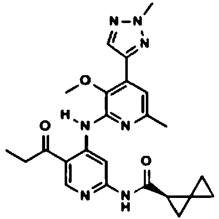 6R |

9. The compound of claim 8, or the pharmaceutically acceptable salt thereof, wherein the compound is Compound 1S, 2S, 3S, 4S, 5S, or 6S in Table B.

10. The compound of claim 8 or the pharmaceutically acceptable salt thereof, wherein the compound is Compound 4S.

11. A pharmaceutical composition, comprising
(i) the compound of claim 1 or the pharmaceutically acceptable salt thereof, and (ii) pharmaceutically acceptable carriers or excipients.

12. A use of the compound of claim 1 or the pharmaceutical composition of claim 5 in the preparation of (i) a drug for treating or preventing a TYK2-mediated disease and/or (ii) a TYK2 inhibitor.

13. The use of claim 12, wherein the TYK2-mediated disease includes: psoriasis, lupus erythematosus, inflammatory bowel disease, psoriatic arthritis, arthritis, vasculitis, fibrosis, dermatitis, skin aging, cephalitis, lupus nephritis, neurogenic inflammation, multiple sclerosis (including optic neuritis, ophthalmoneuromyelitis), chronic inflammatory demyelinating polyneuropathy, Parkinson, dementia, Amyotrophic Lateral Sclerosis, myasthenia gravis, mental disease, schizophrenia, epilepsy, Spinal injury, Sleep disorder, cerebral injury, stroke, Neuropsychiatric Systemic Lupus Erythematosus, diabetic encephalopathy, Sepsis associated encephalopathy, Central Nervous System Neoplasms, Huntington's disease, Neurological syndrome after surgery, pain, itching, depressive disorder, Hypersomnia, hydrocephalus, Ankylosing Spondylitis, respiratory disease, diabetes, Inflammatory eye disease, hepatitis, cardiovascular disease, systemic sclerosis, organ transplantation, alopecia areata, acne, eczema, leucoderma, sjogren syndrome, viral inflammation, cancer, or combinations thereof.

14. A preparation method of a compound, wherein the compound is a compound of formula I-S, 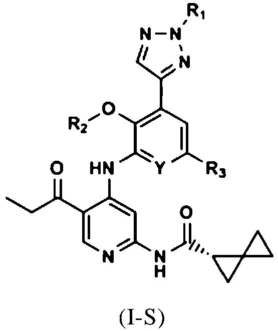 and the preparation method comprises a step of:
reacting an intermediate of formula II-S with an intermediate of formula III, thereby obtaining the compound of formula I-S; 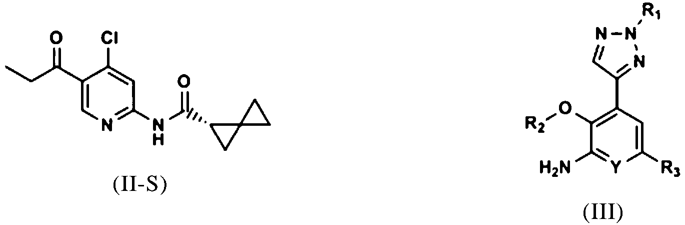
in each formula, Y, R₁, R₂ and R₃ are as defined in formula I.

15. An intermediate of formula II-S,
